# EUROPEAN PATENT APPLICATION

(11) **EP 4 424 270 A1**
(43) Date of publication of application: **04.09.2024**
(21) Application number: 21962542.3
(22) Date of filing: 10.11.2021
(51) Int. Cl.: A61C 8/00, A61C 8/02, A61C 1/00

(54) **OSTEOTOME FOR DENTAL TREATMENT, HOLE FORMATION INSTRUMENT, TEST POST, STOPPER EXTENSION, PERIODONTAL LIGAMENT GUARD, WATER FLOW TUBE, AND MODEL FOR PRE-PROCEDURAL VERIFICATION OF DENTAL TREATMENT PLAN**

(30) Priority: 25.10.2021 JP 2021173960
(71) Applicant: Lookin Corporation, Tokyo 142-0062 (JP)
(72) Inventor: YOSHIHASHI, Noriaki, Tokyo 142-0062 (JP)
(74) Representative: Meissner Bolte Partnerschaft mbB
(86) International application number: PCT/JP2021/041362
(87) International publication number: WO 2023/074005

(57) **Abstract**

In view of various problems when a treatment that embeds an implant body or a treatment that embeds an embedded body such as an autologous tooth is performed, an osteotome for a dental treatment corresponding thereto or the like is provided. To implement this, an osteotome according to the present disclosure is an osteotome 60 used as an instrument which is embedded from a surface layer of a treatment area in a jaw region of a patient into a deep portion to apply an impact in expanding or compressing a bone so as to displace a position of a mucosa behind the treatment area and includes a body portion corresponding to a main body portion of the osteotome and an end portion 63 formed at a distal end of the body portion to displace the position of the mucosa. A diameter of the end portion 63 has a size appropriate for transplant of an autogenous tooth.

## Description

### Technical Field

The present invention relates to an osteotome for a dental treatment, a hole formation instrument, a test post, a stopper extension, a periodontal ligament guard, a water flow tube, and a model for pre-procedural verification of a dental treatment plan.

### Background Art

For example, in an implant treatment, a dentist, who is an operator, normally uses a dental CT scan to obtain three-dimensional image data of an upper jaw and/or a lower jaw, including a treatment position of a patient. On the basis of the three-dimensional image data, the dentist recognizes, prior to a procedure, a position of a maxillary sinus and positions of a posterior superior alveolar artery and a greater palatine artery in the upper jaw of the patient or positions of an inferior alveolar artery and an inferior alveolar nerve in the lower jaw of the patient, in addition to the treatment position.

In a case where, e.g., a treatment target is an upper jaw in an implant treatment, the dentist performs a procedure such that a distal end of a hole formation instrument, such as a drill, does not reach a maxillary sinus mucosa, the posterior superior alveolar artery, the greater palatine artery, and the like of the patient as predetermined not-to-be-reached (not-to-be-contacted) targets. In a case where the treatment target is the lower jaw, the dentist performs a procedure such that the distal end of the hole formation instrument, such as the drill, does not reach the inferior alveolar artery and an inferior alveolar nerve of the patient as predetermined not-to-be-reached targets.

For example, in the implant treatment, a surgical guide that guides the drill may be used to form a concave hole in a bone exactly according to the treatment plan. The surgical guide is formed on the basis of the three-dimensional image data of the upper jaw and/or lower jaw of a patient. The surgical guide is used by being fitted to teeth and a gum of the patient. The surgical guide enables the dentist to form, during the procedure by the dentist, a hole for embedding an implant body such that a position, a direction, a diameter, and the like of the hole, while the hole is formed, are exactly according to the treatment plan.

### Citation List

### Patent Document

Patent Document 1: Japanese Translation of PCT Application No. JP-T-2017-508595

### Summary

### Technical Problem

For example, a risk in the implant treatment is that positions of the not-to-be-reached targets, such as arteries and nerves, cannot be visually recognized during the procedure.

Currently, creation of a treatment plan for an implant treatment (including formation of a surgical guide) is mostly performed by a technician of an implant manufacturer using, e.g., a system of the manufacturer on the basis of CT image data (DICOM data) of a patient acquired by a dentist. The dentist examines the treatment plan created by the technician of the manufacturer. However, the dentist is not on site at which the treatment plan is created, and tools available to the dentist during correction of the treatment plan created by the manufacturer are limited. Since a target is three-dimensional, it is difficult for the dentist to give detailed instructions for the treatment plan, such as selection of an implant body, slight adjustment of an inner diameter, a position, an angle, or the like of a concave hole in which the implant body is to be embedded, and selection of instruments as intended by the dentist.

Ideally, the dentist should be responsible for the implant treatment from the creation of the treatment plan to a real treatment. When there is a mistake in, e.g., selection of various parameters of a hole formation instrument in the treatment plan created by the technician of the manufacturer or the like, it is difficult to eliminate the possibility that the dentist will unconsciously perform the procedure according to the incorrect treatment plan.

It is an object of the present disclosure to provide a pre-procedural verification system for a dental treatment plan, a pre-procedural verification program for a dental treatment plan, a method of producing a model for pre-procedural verification of a dental treatment plan, and a model for pre-procedural verification of a dental treatment plan which allow, when, e.g., a treatment that embeds an implant body or a treatment that embeds an embedded body such as an autologous tooth is to be performed, a dentist who is an operator to preliminarily verify, after the creation of a treatment plan and before a real dental treatment using a procedure instrument such as a hole formation instrument, a positional relationship between a distal end position of the procedure instrument and a not-to-be-reached target position such as a position of a maxillary sinus mucosa, a posterior superior alveolar artery, or a greater palatine artery in an upper jaw of a patient or a position of an inferior alveolar artery or an inferior alveolar nerve in a lower jaw when the procedure instrument is used and allow the dentist to personally perform design, instrument selection, and verification of safety of the treatment plan as much as possible and implement an optimum treatment plan. In addition, in view of various problems when the treatment that embeds the implant body or the treatment that embeds the embedded body such as the autologous tooth is performed, it is also an object of the present disclosure to provide an osteotome for a dental treatment, a hole formation instrument, a test post, a stopper extension, a periodontal ligament guard, a water flow tube, and a model for pre-procedural verification of a dental treatment plan each corresponding thereto.

### Solution to Problem

An osteotome according to an aspect of the present invention is an osteotome used as an instrument which is embedded from a surface layer of a treatment area in a jaw region of a patient into a deep portion to apply an impact in expanding or compressing a bone so as to displace a position of a mucosa behind the treatment area, the osteotome including: a body portion corresponding to a main body portion of the osteotome; and an end portion formed at a distal end of the body portion to displace the position of the mucosa, a diameter of the end portion having a size appropriate for transplant of an autogenous tooth.

In the osteotome described above, the diameter of the end portion may be larger than a diameter of an end portion of an implant-dedicated osteotome.

In the osteotome described above, the diameter of the end portion may be 5 mm or more.

In the osteotome described above, a guided portion which is guided along a guide hole formed in a surgical guide may be formed in a portion of the body portion, the surgical guide being provided according to a surface shape of the treatment area of the patient to offset, by a predetermined amount, a reference position serving as a guide for a depth during a treatment.

Another aspect of the present invention is an osteotome used as an instrument which is embedded, from a proximal end side to a distal end side, in a treatment area in a jaw region of a patient to apply an impact in expanding or compressing a bone so as to displace a position of a mucosa behind the treatment area, the osteotome including: a body portion corresponding to a main body portion of the osteotome; and an end portion formed at a distal end of the body portion to displace the position of the mucosa, a guided portion which is guided along a guide hole formed in a surgical guide being formed in a portion of the body portion, the surgical guide being provided according to a surface shape of the treatment area of the patient to offset, by a predetermined amount, a reference position serving as a guide for a depth during a treatment.

An aspect of the present invention is a hole formation instrument including: a drill that is moved from a surface layer of a treatment area in a jaw region of a patient toward a deep portion to form a hole in which a portion of an embedded body is to be embedded, a diameter of the drill having a size appropriate for transplant of an autogenous tooth.

In the hole formation instrument described above, the diameter of the drill may be larger than a diameter of a drill when the embedded body is an implant body.

In the hole formation instrument described above, the diameter of the drill may be 5 mm or more.

In the hole formation instrument described above, a guided portion which is guided along a guide hole provided in a surgical guide may be formed in a portion of the hole formation instrument, the surgical guide being provided according to a surface shape of the treatment area of the patient to offset, by a predetermined amount, a reference position serving as a guide for a height during a treatment.

An aspect of the present invention is a test post for image diagnosis, formed of a resin material or a ceramic material and inserted into a hole formed in a treatment area in a jaw region of a patient to allow a portion of an embedded body to be embedded therein or into a guide hole formed in a surgical guide to form an image in an image photographed in a state of the insertion by using a radioactive ray and indicate a position of the hole or the guide hole, the surgical guide being provided according to a surface shape of the treatment area of the patient to offset, by a predetermined amount, a reference position serving as a guide for a depth during a treatment.

The test post described above may have a stepped shape.

The test post described above may have an outer diameter portion having a diameter larger than an inner diameter of the guide hole of the surgical guide.

An aspect of the present invention is a stopper extension formed into a cylindrical shape and inserted into a guide hole formed in a surgical guide to extend a guide length, the surgical guide being provided according to a surface shape of a treatment area of a patient to offset, by a predetermined amount, a reference position serving as a guide for a depth during a treatment.

The stopper extension described above may have a stepped shape.

The stopper extension described above may have an outer diameter portion having a diameter larger than an inner diameter of the guide hole of the surgical guide.

An aspect of the present invention is a periodontal ligament guard which partially or entirely covers a portion of an autogenous tooth to be subjected to transplant, except for a tooth root to be cut, to reduce a damage that is possibly be given, by an external cause, to a periodontal membrane located around the tooth root when the tooth root is cut.

The periodontal ligament guard described above may be formed of a low resilience material.

The periodontal ligament guard described above may be made of silicon.

An aspect of the present invention is a water flow tube for transplant, which is embedded from a surface layer of a treatment area in a jaw region of a patient toward a deep portion to jet water from a distal end portion thereof toward a position of a mucosa behind the treatment area, a diameter of the distal end portion having a size appropriate for transplant of an autogenous tooth.

In the water flow tube described above, the distal end portion may be formed with a stepped portion having an outer diameter increasing stepwise, with a distance from a distal end toward a proximal end gradually increasing .

An another aspect of the present invention is a model for pre-procedural verification of a dental treatment plan, the model including: a model main body formed on the basis of a three-dimensional image of a jaw region of a patient to have the same size and shape as those of at least a portion of each of a tooth of the patient to be treated, a gum thereof, and an alveolar bone thereof covered with the gum and imitate at least a portion of each of the tooth of the patient to be treated, the gum thereof, and the alveolar bone thereof; a target model portion formed on the basis of the three-dimensional image of the patient to be adjacent to or embedded in the alveolar bone of the treatment target of the patient, imitate a not-to-be-contacted target with which a distal end of a procedure instrument selected from among a plurality of the procedure instruments is to be kept from contact during a treatment, and be provided in the model main body to have the same positional relationship as a positional relationship between each of the tooth of the treatment target of the patient, the gum thereof, and the alveolar bone and the not-to-be-contacted target; and a hole portion formed, on the basis of the three-dimensional image of the patient, at a position in the model main body imitating the gum and alveolar bone of the treatment target of the patient to imitate a hole which allows an embedded body to be embedded in the treatment target and allow a dentist to visually recognize a positional relationship between the procedure instrument and the target when the procedure instrument in the three-dimensional image of the patient is fitted in the hole in the three-dimensional image and the procedure instrument is inserted into the hole in the three-dimensional image, a fluid for making a notification of, when the procedure instrument comes into contact with the target model portion during pre-procedural verification, the contact being embedded therein.

With such a model, during pre-procedural verification of a dental treatment plan, when a procedure instrument comes into contact with a target model portion imitating a not-to-be-contacted target, a fluid (such as a colored liquid) leaks out, and therefore it is possible to easily visually recognize the contact with the target model portion.

### Brief Description of Drawings

Fig. 1 is a block diagram of a pre-verification system for a dental treatment plan according to a first embodiment and a second embodiment.
Fig. 2 is a schematic diagram illustrating an example of an implant body.
Fig. 3 is a schematic diagram illustrating an example of a hole formation instrument.
Fig. 4A is a schematic diagram illustrating a model for pre-variation of a dental treatment plan according to the first embodiment.
Fig. 4B is a schematic diagram illustrating the model viewed from a direction different from that in Fig. 4A.
Fig. 4C is a schematic diagram illustrating a model for pre-verification of a dental treatment plan according to a modification of the first embodiment.
Fig. 5 is a schematic diagram illustrating a surgical guide according to the first embodiment.
Fig. 6 is a flow chart illustrating a treatment plan for an implant treatment.
Fig. 7 is a flow chart illustrating the treatment plan for the implant treatment continued from Fig. 6.
Fig. 8 is a diagram illustrating a state where third three-dimensional image data obtained by marking positions of not-to-be-reached targets is superimposed on first three-dimensional image data representing an inferior alveolar bone.
Fig. 9 is a diagram illustrating a state where fourth three-dimensional image data of the implant body is further superimposed on Fig. 8.
Fig. 10 is a diagram illustrating a state where second three-dimensional image data of teeth and a gum and fifth three-dimensional image data of the hole formation instrument are further superimposed on Fig. 9.
Fig. 11 is a diagram illustrating sixth three-dimensional image data of the surgical guide.
Fig. 12 is a schematic diagram illustrating the gum, the teeth, the inferior alveolar bone, the implant body, and the not-to-be-reached target in a state where the surgical guide is attached to a lower jaw.
Fig. 13 is a schematic diagram illustrating the gum, the teeth, the inferior alveolar bone, the hole formation instrument, and the not-to-be-reached target in the state where the surgical guide is attached to the lower jaw.
Fig. 14 is a diagram illustrating a state where the sixth three-dimensional image data of the surgical guide is further superimposed on Fig. 9.
Fig. 15 is a diagram illustrating a state where the first three-dimensional image data and the second three-dimensional image data has been removed from Fig. 14.
Fig. 16 is a diagram illustrating surface image data of a portion of each of the second three-dimensional image data, the third three-dimensional image data, the fourth three-dimensional image data, and the fifth three-dimensional image data.
Fig. 17 is a diagram illustrating a surface image obtained by superposing the surface image data illustrating Fig. 16, and then subtracting the fourth three-dimensional image data and the fifth three-dimensional image data therefrom.
Fig. 18 is a schematic diagram illustrating a state where a model is combined with the surgical guide, and then the hole formation instrument is inserted in a guide hole of the surgical guide.
Fig. 19 is a schematic diagram illustrating the gum, the teeth, the inferior alveolar bone, the hole formation instrument including a handle, and the not-to-be-reached target in a state where the surgical guide is attached to the lower jaw according to a first modification.
Fig. 20 is a diagram illustrating a state where the third three-dimensional image data obtained by marking the position of the not-to-be-reached target, the fourth three-dimensional image data of an autologous tooth, and fifth three-dimensional image data of the hole formation instrument is superimposed on the first three-dimensional image data representing the inferior alveolar bone.
Fig. 21 is a diagram according to the second embodiment which illustrates a state where the fourth three-dimensional image data of the implant body and the fifth three-dimensional image data of the hole formation instrument are superimposed on the first three-dimensional image data representing a superior alveolar bone, a maxillary sinus, a posterior superior alveolar artery, and a greater palatine artery in an upper jaw.
Fig. 22 is a diagram illustrating a state where the first to fourth three-dimensional image data is superposed.
Fig. 23 is a diagram illustrating a state where the sixth three-dimensional image data is superimposed on the diagram illustrated in Fig. 22, while the first three-dimensional image data has been removed therefrom.
Fig. 24 is a diagram illustrating surface image data of a portion of each of the first three-dimensional image data, the second three-dimensional image data, the third three-dimensional image data, the fourth three-dimensional image data, and the fifth three-dimensional image data.
Fig. 25 is a diagram illustrating a surface image obtained by superposing the surface image data illustrated in Fig. 24, and then subtracting the fourth three-dimensional image data and the fifth three-dimensional image data therefrom.
Fig. 26 is a diagram obtained by viewing the upper jaw illustrated in Fig. 25 in a different direction.
Fig. 27 is a schematic diagram illustrating a positional relationship between the teeth and the maxillary sinus when a procedure of embedding the implant body in the upper jaw is performed.
Fig. 28 is a schematic diagram illustrating the positional relationship between the teeth and the maxillary sinus when the procedure of embedding the implant body in the upper jaw is performed, which is subsequent to Fig. 27.
Fig. 29 is a diagram illustrating a state where a maxillary sinus floor mucosa of the maxillary sinus is superimposed on the second three-dimensional image data in Fig. 22.
Fig. 30A is a diagram illustrating an example of an osteotome in the third embodiment.
Fig. 30B is a diagram illustrating a portion of the osteotome in enlarged relation.
Fig. 30C is a partially enlarged view of the osteotome in a state where a bar portion is displaced to a proximal end side.
Fig. 31 is a cross-sectional view along a line XXXI-XXXI in Fig. 30B.

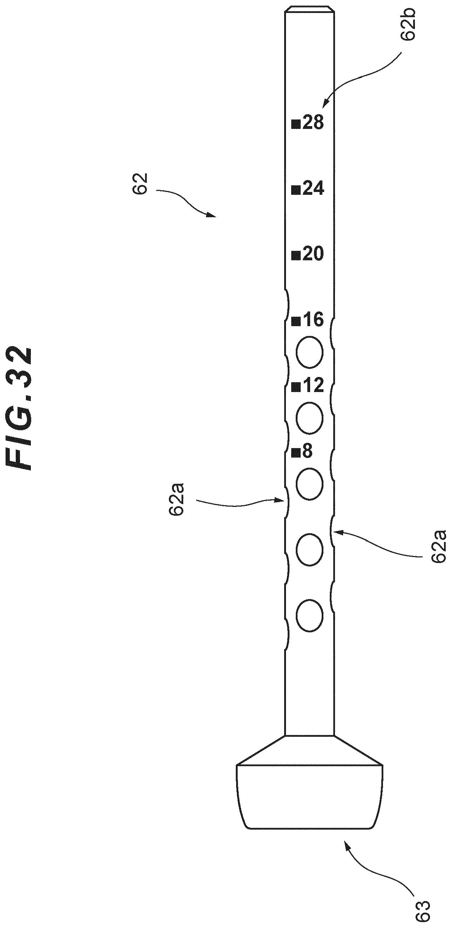

Fig. 32 is a diagram illustrating an example of the bar portion having an end portion with a large diameter.
Fig. 33A is a diagram illustrating a state where the surgical guide is fitted to the teeth and gum in the vicinity of a treatment area of a jaw region of a patient.
Fig. 33B is a diagram illustrating formation of a concave hole in the treatment area with the hole formation instrument.
Fig. 33C is a diagram illustrating a state where the concave hole is formed, and then the hole formation instrument is pulled out.
Fig. 33D is a diagram illustrating formation of a larger concave hole in the treatment area with a larger-diameter hole formation instrument.
Fig. 33E is a diagram illustrating a state where the larger concave hole is formed, and then the hole formation instrument is pulled out.
Fig. 34A is a diagram illustrating a state where the surgical guide is fitted again, and the osteotome is inserted in the guide hole of the surgical guide.
Fig. 34B is a diagram illustrating insertion of an end portion of the osteotome into a surface layer of the larger concave hole formed in the treatment area for displacing a position of a mucosa behind the treatment area.
Fig. 34C is a diagram illustrating a state where the position of the mucosa is displaced, and then the osteotome and the surgical guide are detached.
Fig. 34D is a diagram illustrating a state where an osteotome having an end portion with a large diameter is attached together with the surgical guide.
Fig. 34E is a diagram illustrating further displacement of the position of the mucosa behind the treatment area using the osteotome having the end portion with the large diameter.
Fig. 34F is a diagram illustrating a state where the position of the mucosa is further displaced, and then the osteotome and the surgical guide are detached.
Fig. 34G is a diagram illustrating a state where the osteotome having the end portion with the large diameter is attached together with the surgical guide.
Fig. 34H is a diagram illustrating further displacement of the position of the mucosa behind the treatment area using the osteotome having the end portion with the large diameter.
Fig. 35A is a diagram illustrating a state where the position of the mucosa is further displaced, and then the osteotome and the surgical guide are detached.
Fig. 35B is a diagram illustrating a state where the position of the mucosa is further displaced, and then a water flow tube is attached together with the surgical guide.
Fig. 35C is a diagram illustrating displacement of the position of the mucosa using water jetted from the water flow tube.
Fig. 35D is a diagram illustrating further displacement of the position of the mucosa using the water jetted from the water flow tube.
Fig. 35E is a diagram illustrating a state where an autogenous tooth is transplanted in the concave hole after the further displacement of the position of the mucosa using the water jetted from the water flow tube.
Fig. 36A is an image representing a real example in which a position of an embedded body is displaced.
Fig. 36B is an image representing an example of a test post.
Fig. 36C is a diagram illustrating another example of the test post.
Fig. 36D is a diagram illustrating an example of the surgical guide actually produced on the basis of image data.
Fig. 36E is a diagram illustrating the surgical guide to which the test post is attached.
Fig. 36F is a diagram illustrating an example of a CT image obtained by photographing the surgical guide to which the test post is attached and which is attached the patient.
Fig. 37A is a diagram illustrating an example of a stopper extension.
Fig. 37B is a diagram illustrating the stopper extension attached to the handle and holding a shank of the hole formation instrument.
Fig. 37C is a diagram illustrating a state where a portion of the stopper extension attached to the handle and holding the shank of the hole formation instrument is inserted in the guide hole of the surgical guide.
Fig. 38A is a diagram illustrating, for reference, an example of the autogenous tooth before covered with a periodontal ligament guard.
Fig. 38B is a diagram illustrating the autogenous tooth in a state covered with the periodontal ligament guard.
Fig. 38C is a diagram illustrating the autogenous tooth in a state covered with the periodontal ligament guard, which is held between fingers.

### Description of Embodiments

A pre-procedural verification system for a dental treatment plan (a model production system for verifying the dental treatment plan after creation of the dental treatment plan and before a procedure) 10 assumes a part of a sequence of tasks from, e.g., acquiring various data of a given patient and creating a treatment plan for an implant treatment to performing a procedure on the patient. The system 10 is used when, e.g., the sequence of tasks from, e.g., acquiring affected area data from a given patient and creating a dental treatment plan for the given patient to performing pre-procedural verification of the dental treatment plan using an output real patient model (model for the pre-procedural verification of the dental treatment plan) 100 are performed. The acquisition of the affected area data from the given patient to the outputting of the model 100 may also be performed using another system different from the system 10.

### (First Embodiment)

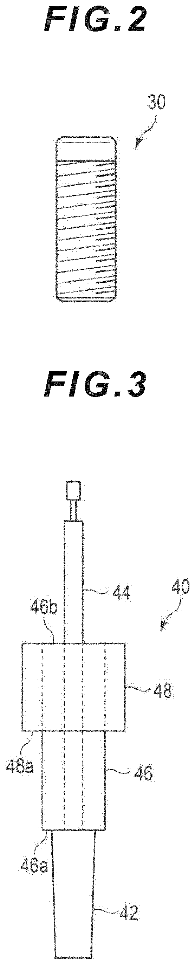

In the first embodiment, a description will be given of a case where the system 10 is used for an example in which an implant treatment is performed by embedding an implant body 30 (see Fig. 2) as an embedded body selected from among a large number of embedded bodies in a lower jaw of a patient.

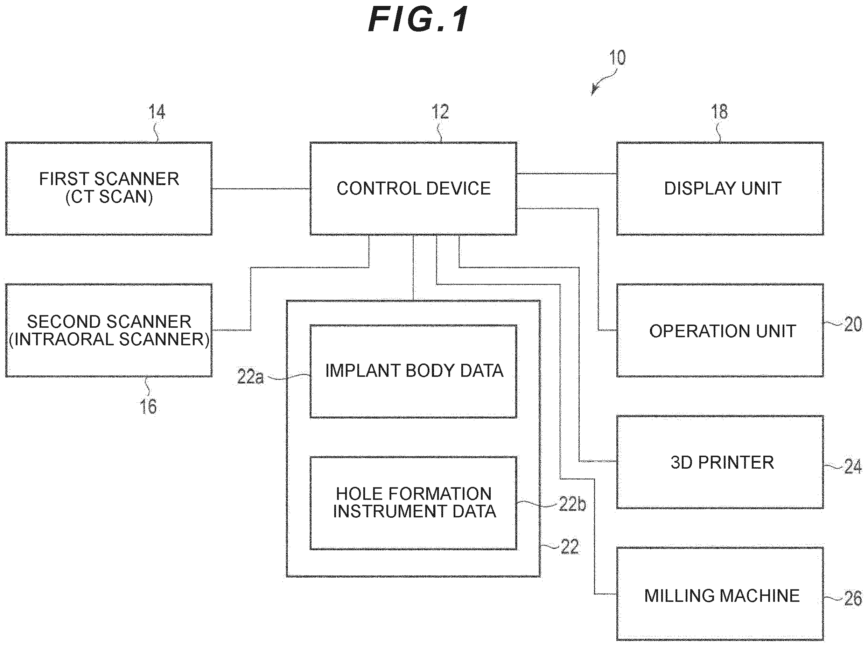

As illustrated in Fig. 1, the pre-procedural verification system for a dental treatment plan (hereinafter referred to simply as the system) 10 includes a control device 12, a first scanner 14, a second scanner 16, a display unit 18, an operation unit (instruction input unit) 20, a storage device 22, a 3D printer 24, and a milling machine 26.

The control device 12 controls the first scanner 14, the second scanner 16, the display unit 18, the operation unit 20, the storage device 22, the 3D printer 24, and the milling machine 26. As the control device 12, e.g., a computer is used. The control device 12 includes a processor such as, e.g., a CPU or MPU, a RAM, a ROM, and an I/O interface. In the control device 12, the processor such as, e.g., the one or plurality of CPUs develops, in the RAM, a control program stored in the memory such as the ROM and executes appropriate processing on the display unit 18, the operation unit 20, the storage device 22, the first scanner 14, the second scanner 16, the 3D printer 24, and the milling machine 26. Alternatively, in the control device 12, the processor such as, e.g., the one or plurality of CPUs read a program via a network, and executes appropriate processing on the display unit 18, the operation unit 20, the storage device 22, the first scanner 14, the second scanner 16, the 3D printer 24, and the milling machine 26. The control device 12 implements, using software, a function of controlling each of the units and performing processing such as image processing through reading and execution of a program stored in advance in the memory by the processor.

The first scanner 14 is, e.g., a dental CT scan. The first scanner 14 acquires three-dimensional image data (e.g., DICOM data) of, e.g., teeth, a bone, and the inside of the bone in the lower jaw of the patient, and outputs the three-dimensional image data to the control device 12. The control device 12 causes the storage device 22 to store the three-dimensional image data of the teeth, the bone, and the inside of the bone in the lower jaw of the patient. The second scanner 16 is, e.g., an intraoral scanner. The second scanner 16 acquires three-dimensional image data (e.g., STL data) of, e.g., the teeth and a gum surface in the lower jaw of the patient, and outputs the three-dimensional image data to the control device 12. The control device 12 causes the storage device 22 to store the three-dimensional image data of the teeth and the gum surface in the lower jaw of the patient.

The display unit 18 is one of various displays such as, e.g., a liquid crystal display and an organic EL display. The display unit 18 displays images related to the patient acquired by the first scanner 14 and the second scanner 16 and output to the control device 12, while displaying various information. The control device 12 may also read the three-dimensional image data of the patient stored in the storage device 22, and cause the display unit 18 to display the three-dimensional image data.

The operation unit 20 inputs instructions to the control device 12. The operation unit 20 includes devices such as, e.g., a keyboard and a mouse.

The storage device 22 stores various data of the patient (e.g., the three-dimensional image data (DICOM data) of the teeth, the bone, and the inside of the bone in the lower jaw and the three-dimensional image data (e.g., STL data) of the teeth and the gum surface in the lower jaw of the patient). The storage device 22 stores, e.g., various three-dimensional image data (implant body data) 22a of the implant body 30 illustrated in Fig. 2 and various three-dimensional image data (hole formation instrument set data) 22b of a hole formation instrument 40, such as a drill illustrated in Fig. 3.

When the control device 12 reads the various three-dimensional image data 22a of the implant body 30 and the various three-dimensional image data 22b of the hole formation instrument 40 via a network, it may be no longer necessary to cause the storage device 22 to store the various three-dimensional image data 22a of the implant body 30 and the various three-dimensional image data 22b of the hole formation instrument 40. In other words, the control device 12 may also use, e.g., a database (the implant body data 22a and the hole formation instrument set data 22b) on a server, instead of the storage device 22. The control device 12 can read the various data from the server.

As illustrated in Fig. 3, the real hole formation instrument 40 has, e.g., a body 42 that makes a hole, a shank 44, a sleeve 46, and a stopper 48. The body 42 and the shank 44 are integrated by casting or the like. The shank 44 is fixed to a hand piece not shown. Accordingly, the body 42 and the shank 44 rotate around an axis of a predetermined rotation shaft. The sleeve 46 covers the outside of the body 42 to be fitted into or engaged with a guide hole (through hole) 210 of an auxiliary instrument 200 such as a surgical guide described later. The stopper 48 has an end surface 48a on the body 42 side which comes into contact with a defining surface 220 that defines the guide hole 210 of the auxiliary instrument 200 in a state where, e.g., a distance to the hand piece is regulated.

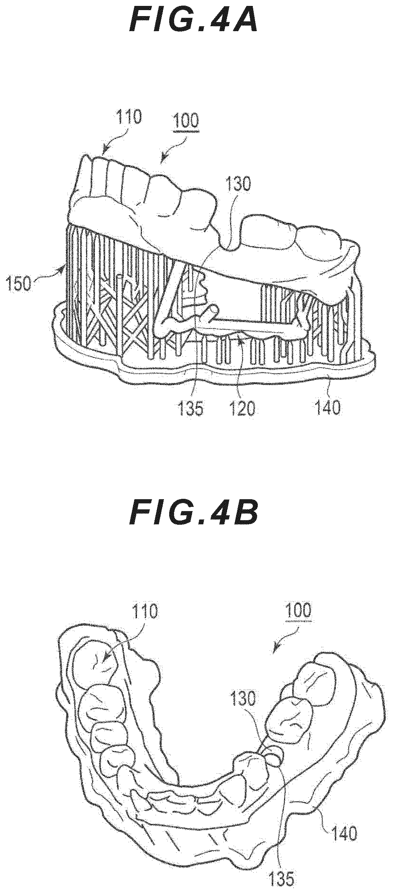

The 3D printer 24 forms a model 100 for pre-dental-treatment verification illustrated in Figs. 4A and 4B on the basis of each of the three-dimensional image data of the lower jaw of the patient and three-dimensional image data produced by a dentist. The 3D printer 24 is preferably owned by, e.g., the dentist and operated by the dentist, but a professional company or the like may also receive data for the 3D printer from the dentist or the like, and output a modeled object.

The model 100 has a model main body 110, a target model portion 120, and a hole portion 130. The model main body 110 is formed into the same size and shape as those of a treatment target area and the vicinity thereof in the real lower jaw of the patient. The model main body 110 and the target model portion 120 are formed so as to have, therebetween, the same positional relationship as a positional relationship between the treatment target area in the real lower jaw of the patient and a target (not-to-be-contacted target or a to-be-contacted target). The same positional relationship means that a portion of the treatment target area which is related to a procedure is formed into the same size and shape as those of the treatment target area and the vicinity thereof in the real lower jaw of the patient. The hole portion 130 is defined by various parameters of the implant body 30 and the hole formation instrument 40 each described later. The hole portion 130 is formed according to the setting of the parameters related to the shape (e.g., a length or an outer diameter), an angle, and a position of the implant body 30 to be embedded in the hole portion 130 of the treatment target area or the hole formation instrument 40.

The model main body 110 has a defining surface 135 forming an edge portion of the hole portion 130. The defining surface 135 is the surface of a gum or an inferior alveolar bone. When an end surface 46a of the sleeve 46 comes into contact with the defining surface 135, the defining surface 135 restricts the body 42 of the hole formation instrument 40 from moving from that position to a side behind the gum and the inferior alveolar bone. Consequently, a position reached by a distal end of the hole formation instrument 40 is restricted by the defining surface 135 of the model main body 110.

Preferably, the target model portion 120 is supported by the model main body 110. The model 100 further has a base portion (basis) 140 that supports the target model portion 120 and is provided opposite to the model main body 110 and braces 150 connecting the model main body 110 and the base portion 140.

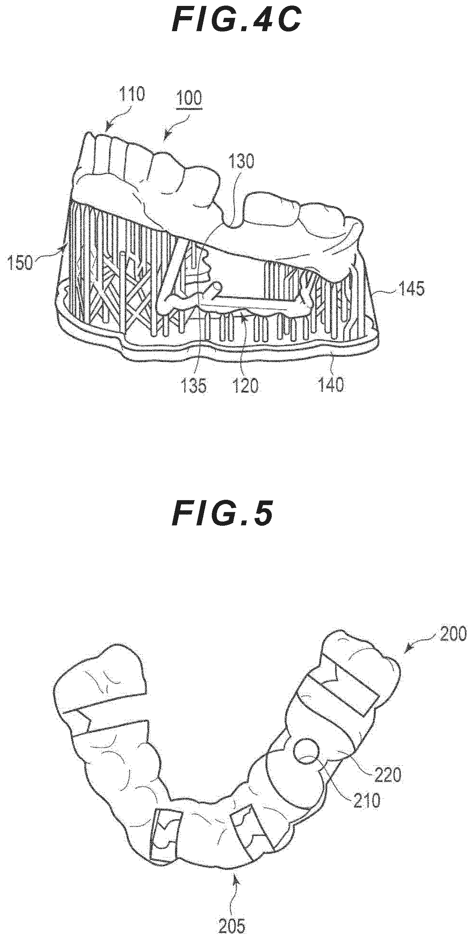

The milling machine 26 carves out the auxiliary instrument (surgical guide) 200 illustrated in Fig. 5 on the basis of each of the three-dimensional image data of the lower jaw of the patient and the three-dimensional image data produced by the dentist. The milling machine 26 is preferably owned by, e.g., the dentist and operated by the dentist, but a professional company or the like may also receive data for the milling machine 26 from the dentist or the like, and output the auxiliary instrument 200.

The auxiliary instrument 200 actually used during a dental treatment uses a resin material that is medically approved and has a durability sufficient to withstand the dental treatment. The auxiliary instrument 200 to be used during the dental treatment may also be produced with the 3D printer 24. The auxiliary instrument 200 in this case is formed of a medically approved material. Note that the auxiliary instrument 200 which is not to be used during the dental treatment, but is to be used together with the model 100 for confirmation by the dentist is preferably formed of the same material as that of, e.g., the model 100. The model 100 is not used for a real treatment, and therefore needs only to be formed of an appropriate material with appropriate precision as long as a relationship between the defining surface 135 and the target model portion 120 is maintained.

The auxiliary instrument 200 is formed according to a surface shape of the treatment area of the patient. The auxiliary instrument 200 is used by being fixed to teeth, a gum, or a jaw bone in the vicinity of a gum in the treatment area of the patient with a screw and then fitted thereto. The auxiliary instrument 200 is fixed to the lower jaw in a state where backlash thereof with respect to the lower jaw is prevented. The auxiliary instrument 200 is used to form a concave hole set to the gum and an alveolar bone and precisely form a concave hole for placing the set implant body 30 into the concave hole with the body 42 of the hole formation instrument 40.

The auxiliary instrument 200 has a main body 205 and the guide hole 210.

As described above, the auxiliary instrument 200 is used by being fixed to the teeth, gum, and jaw bone in the vicinity of the gum in the treatment area of the patient with the screw and then fitted thereto, and therefore the main body 205 is used as a position defining portion that defines a reference position with respect to the teeth, gum, and alveolar bone. In the illustrated example, the main body 205 is fixed along, e.g., one or more teeth in an intraoral region of the patient, but a lower jaw side of the patient may also be in a completely edentulous state. For example, the main body 205 can be configured so as to be connected to a smaller oral portion of the patient, such as only one or two teeth, only a bone, or any combination of any of the portions.

The guide hole 210 defines a direction, a shape (length or outer diameter), an angle, a position, and the like of the concave hole formed by the body 42 of the hole formation instrument 40 when the main body 205 is appropriately attached to the teeth and gum in the lower jaw of the patient. The auxiliary instrument 200 has the defining surface 220 forming the edge portion of the guide hole 210. The defining surface 220 is a surface opposite to a surface facing the gum or inferior alveolar bone. When the end surface 48a of the stopper 48 comes into contact with the defining surface 220, the defining surface 220 restricts the body 42 of the hole formation instrument 40 from moving from that position toward a side behind the gum and the inferior alveolar bone. Accordingly, a position reached by the distal end of the hole formation instrument 40 is defined by the defining surface 220 of the auxiliary instrument 200.

Note that the guide hole 210 of the auxiliary instrument 200 may possibly allow not only the hole formation instrument 40, but also another procedure instrument, such as, e.g., an electric scalpel for stopping bleeding, to be inserted therein. At this time, the restricting surface 220 restricts a direction of insertion, an angle, a position, or the like of the electric scalpel. As a result, the restricting surface 220 can be used not only as the restricting surface for the hole formation instrument 40, but also as a restricting surface for a procedure instrument. Therefore, the auxiliary instrument 200 is used as a position defining body for the procedure instrument.

Note that, in the system 10 according to the present embodiment, the dentist can produce the auxiliary instrument as three-dimensional image data (data representing a three-dimensional shape), and can form a real object conforming to a shape and a size of the lower jaw of the patient by using the 3D printer 24 or the milling machine 26.

In the control device 12 according to the present embodiment, an image display program, an image processing program, an output program, and the like are stored.

The image display program causes the display unit 18 to display the data acquired by the first scanner 14 and the second scanner 16. The image processing program superposes the image data displayed using the image display program and the various data stored in the storage device 22 on the basis of the same coordinate axes. The image display program causes the display unit 18 to display the data superposed on the basis of the same coordinate axes by using the image processing program. In addition, the image display program also causes the display unit 18 to display the three-dimensional image data produced on the basis of an intention of the dentist. The output program outputs the three-dimensional image data produced on the basis of the intention of the dentist by using the image processing program and surface data (which refers to data representing a three-dimensional shape (three-dimensional image data) and is hereinafter referred to as the surface data) of an object (e.g., the model 100 or the auxiliary instrument 200). The output program can output the surface data that causes the 3D printer 24 or the milling machine 26 to form a real object.

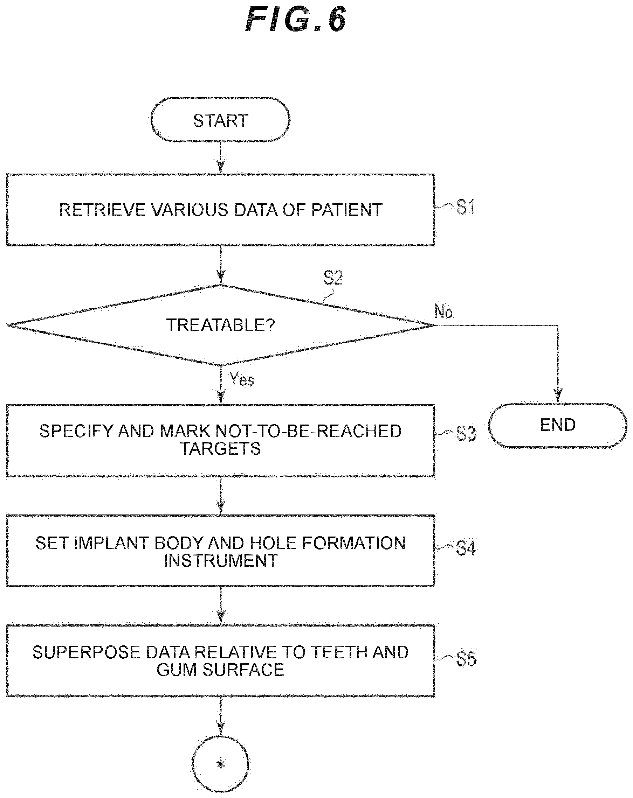

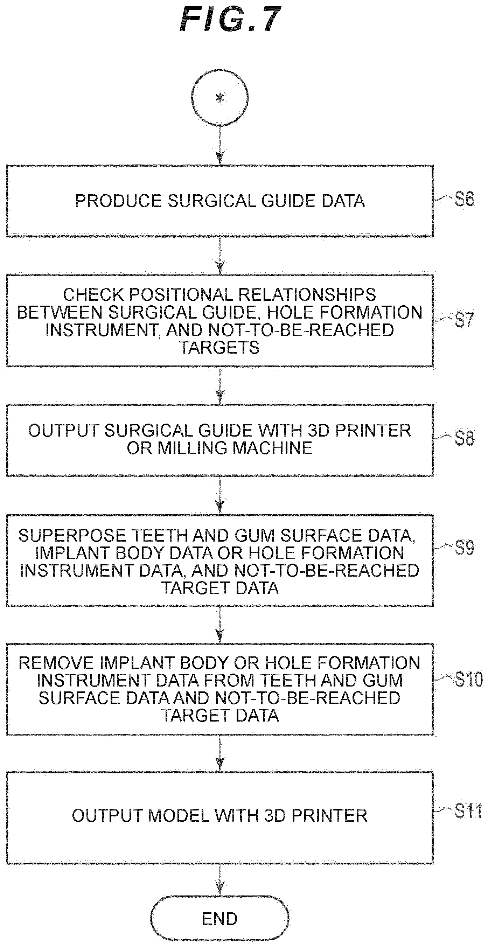

The dentist uses the operation unit 20 to input various instructions to the control device 12, and creates a treatment plan for an implant treatment according to, e.g., a flow illustrated in Figs. 6 and 7.

The dentist uses the first scanner 14 such as a dental CT scan to acquire three-dimensional image data (e.g., DICOM data) of the teeth, the bone, and the inside of the bone in the lower jaw of the patient, and causes the storage device 22 to store the three-dimensional image data. This is assumed to be first three-dimensional image data. In addition, the dentist uses the second scanner 16 such as, e.g., an intraoral scanner to acquire three-dimensional image data (e.g., STL data) of the teeth and the gum surface, and causes the storage device 22 to store the three-dimensional image data. This is assumed to be second three-dimensional image data (first surface data) 52. The dentist retrieves the first three-dimensional image data and the second three-dimensional image data into appropriate software (an application) (Step S1). The software can read both of first three-dimensional image data 51 and the second three-dimensional image data 52 even when respective data formats of the first three-dimensional image data 51 and the second three-dimensional image data 52 are different.

The dentist checks, e.g., the first three-dimensional image 51 based on the first three-dimensional image data on a display screen of the display unit 18, while comprehensively determining whether or not an implant treatment on a treatment area of a given patient is possible on the basis of various other conditions (Step S2). A description will be given below of a task when the dentist determines that the implant treatment is possible (S2-Yes). Note that, when the dentist determines that the implant treatment is impossible (S2-No), the task of creating the treatment plan is ended.

Note that, when the second three-dimensional image data is not used for the determination of whether or not the implant treatment is possible, the acquisition of the three-dimensional image data (e.g., STL data) of the teeth and the gum surface using the second scanner 16 may also be performed after a step in Step S2.

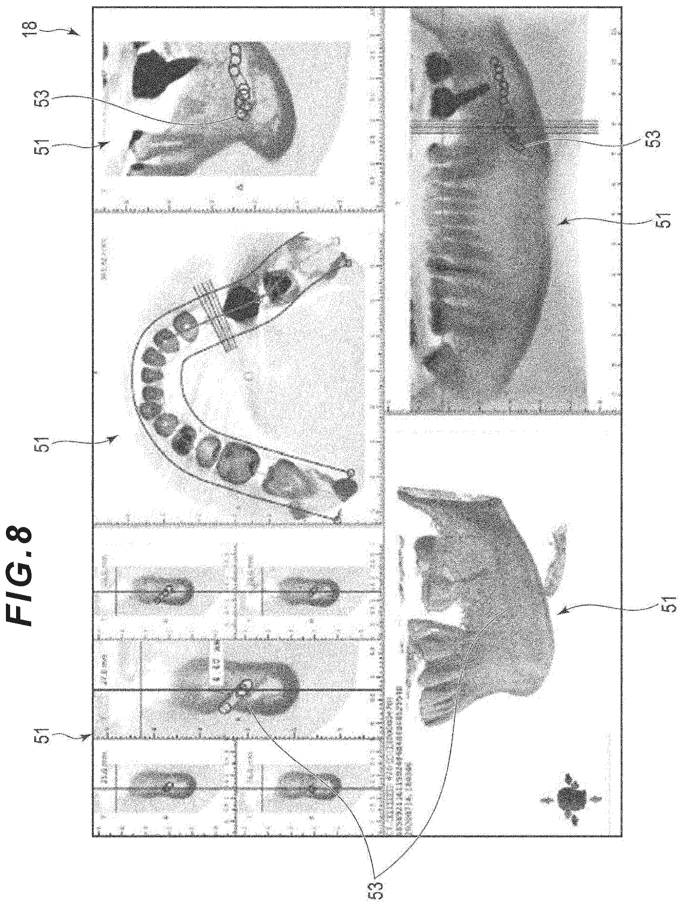

As illustrated in Fig. 8, the dentist specifies, in the software, positions of the inferior alveolar artery and the inferior alveolar nerve in the first three-dimensional image 51 representing the bone in the lower jaw (inferior alveolar bone) to use the specified positions as not-to-be-reached (not-to-be-contacted) targets not to be reached by the distal end of the body 42 of the hole formation instrument 40 when a concave hole 70 in which the implant body 30 is to be embedded during the implant treatment is formed. Then, the dentist marks the positions of the not-to-be-reached targets in the software (Step S3 or first processing). At this time, the dentist marks feature points of the not-to-be-reached targets in the software such that the not-to-be-reached targets are three-dimensionally formed. Then, the dentist produces the not-to-be-reached targets as third three-dimensional image data (fourth surface data) 53.

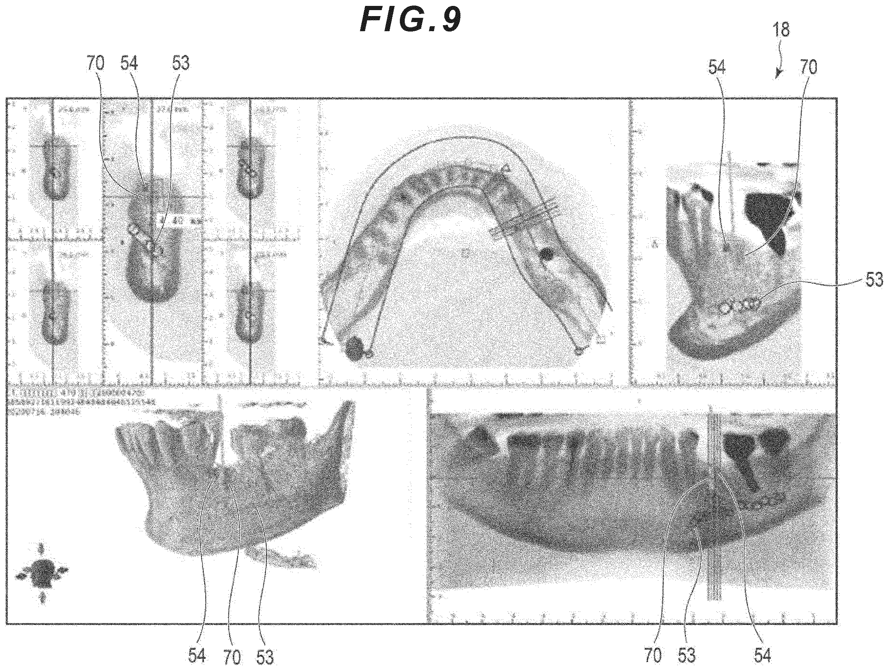

As illustrated in Fig. 9, in the software, the dentist sets or installs the shape (e.g., length or outer diameter), angle, position, and the like of an implant body imitating the implant body 30 at a treatment position (Step S4). The installation means retrieval of, e.g., three-dimensional data acquired by the dentist by using the 3D scanner or the like into the system 10. The angle of the implant body refers to, e.g., an orientation of the implant body with respect to the treatment position of the patient.

Note that, as the implant body, three-dimensional image data corresponding to the implant body in one-to-one relation and including information on the same shape and size as those of the actually medically approved implant body 30 is used. At this time, the dentist considers relationships with shapes of an abutment and an upper structure (tooth crown portion), which are to be attached to the implant body 30. The dentist selects the one implant body compliant with the settings from, e.g., the three-dimensional image data 22a of implant bodies stored in the storage device 22. Fourth three-dimensional image data (second surface data) 54 of the implant body selected by the dentist is provided from, e.g., a manufacturer of the implant body 30. When the fourth three-dimensional image data 54 is not provided from the manufacturer of the implant body 30, the dentist may also personally produce the fourth three-dimensional image data 54 of the implant body. The control device 12 causes the storage device 22 to store the fourth three-dimensional image data 54 of the implant body produced by the dentist.

The dentist can personally select the implant body 30, and optimally set the angle, position, and the like of the implant body 30 with respect to the bone in the lower jaw according to the patient. At this time, the dentist can easily re-select the implant body and test the suitability of the implant body which is different in length and diameter to a procedure target in the patient.

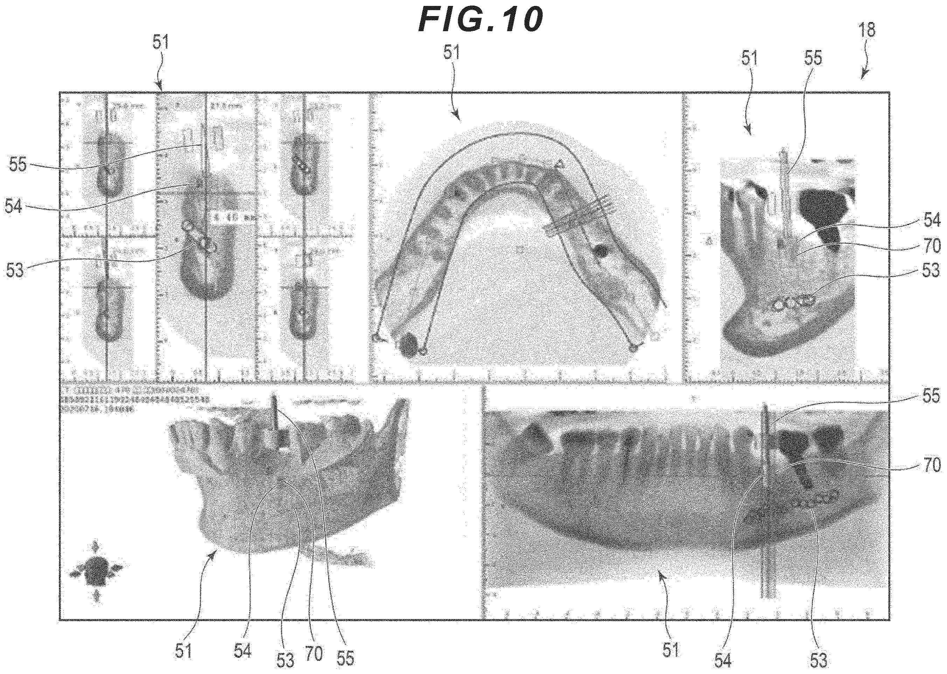

As illustrated in Fig. 10, the dentist selects, from a drill set from one or a plurality of manufacturers to be used for a treatment of the patient, an appropriate hole formation instrument according to a depth, a diameter, an angle, and a position of the concave hole 70 in which the selected implant body is to be embedded (second processing). Note that, as the hole formation instrument, three-dimensional image data corresponding to the hole formation instrument in one-to-one relation and including information on the same shape and size as those of the actually medically approved hole formation instrument 40 is used. The dentist can normally select the hole formation instrument 40 recommended by the manufacturer of the selected implant body 30, but may also use another hole formation instrument 40. The dentist selects the one hole formation instrument 40 matching the settings from the three-dimensional image data 22b of the hole formation instrument imitating the hole formation instrument 40 stored in the storage device 22. Fifth three-dimensional image data (third surface data) 55 of the hole formation instrument 40 selected by the dentist is provided from, e.g., the manufacturer of the hole formation instrument 40. When the fifth three-dimensional image data 55 is not provided from the manufacturer of the hole formation instrument 40, the dentist may also personally produce the fifth three-dimensional image data 55. The control device 12 causes the storage device 22 to store the fifth three-dimensional image data 55 produced by the dentist.

Note that, in general, the hole portion 130 resulting from the real hole formation instrument 40 is formed to have a diameter slightly smaller than an outer diameter of the real implant body 30. This relationship is the same as a relationship in the software. An outer diameter difference between the hole formation instrument and the implant body can appropriately be set by the dentist by inputting the outer diameter difference as a parameter to the operation unit 20.

The dentist aligns, in the software, the respective coordinate axes of the first to fifth three-dimensional image data 51 to 55, and superimposes the second three-dimensional image data 52 on the first three-dimensional image data 51, the third three-dimensional image data 53, and the fourth three-dimensional image data 54 and/or the fifth three-dimensional image data 55 in a predetermined coordinate system with the coincident coordinate axes (Step S5). Consequently, as illustrated in Fig. 10, the dentist clearly shows, on the display unit 18, positional relationships between the teeth and the gum surface in the lower jaw and the artery and the nerve in the alveolar bone by using the software.

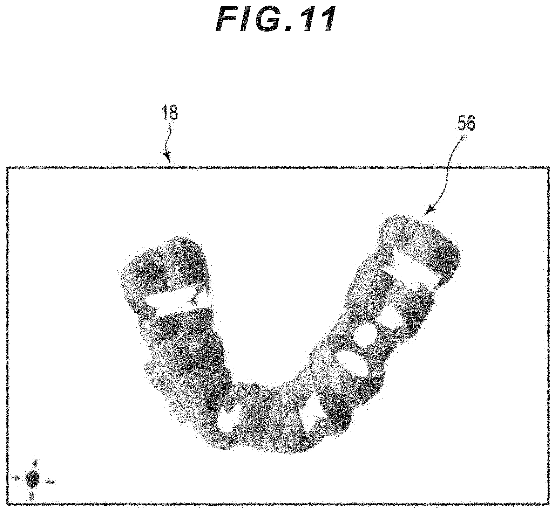

As illustrated in Fig. 11, the dentist produces, in the software displayed on the display unit 18, the three-dimensional image data of the auxiliary instrument (surgical template) according to the shape of the lower jaw of the patient (Step S6). In other words, the dentist produces the auxiliary instrument as a sixth three-dimensional image 56.

The auxiliary instrument 200 illustrated in Fig. 5 guides the body 42 of the hole formation instrument 40 in the treatment area, while covering the gum. When determining the treatment plan, the dentist outputs three-dimensional image data (e.g., STL data) for the 3D printer 24 or the milling machine 26 of the auxiliary instrument 200, and forms the auxiliary instrument 200 with the 3D printer 24 or the milling machine 26. The auxiliary instrument 200 can be fitted to or engaged with the model 100 (see Figs. 4A and 4B) produced with the 3D printer 24. Note that the resin material to be used for the auxiliary instrument 200 differs depending on whether or not the auxiliary instrument 200 is to be actually used during the treatment. When a medically approved resin material is used as the auxiliary instrument 200, the auxiliary instrument 200 can be used without alteration. When a medically unapproved resin material is used as the auxiliary instrument 200, this auxiliary instrument 200 cannot be used without alteration for a real treatment. In this case, as will be described later, the auxiliary instrument 200 is used to verify the created treatment plan before a procedure in such a manner as to confirm that, in a state where the auxiliary instrument 200 is fitted to the model 100, the distal end of the hole formation instrument 40 is placed at a predetermined position and does not come into contact with the target model portion 120.

It is to be noted herein that, in the real procedure, the dentist cannot check how many millimeters the gum (not the alveolar bone) is being cut down from the surface thereof during formation of the concave hole in the treatment target area of the patient, but can check in advance how many millimeters the alveolar bone is being cut down from the surface thereof or a distance from the alveolar bone surface to the artery by using a tool to be actually used.

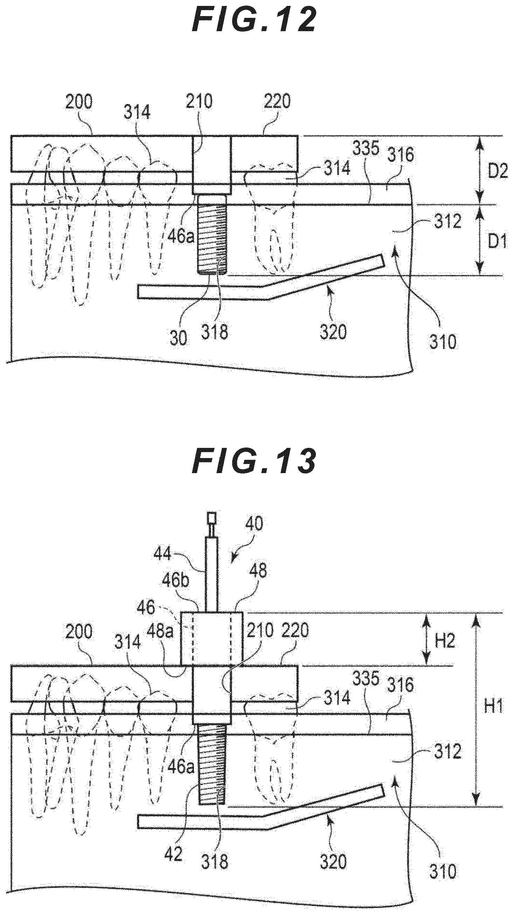

Figs. 12 and 13 illustrate schematic diagrams of a lower jaw 310 including an alveolar bone 312, teeth 314, a gum 316, a concave hole 318, and a not-to-be-reached target 320 of the inferior alveolar artery and the inferior alveolar nerve.

As illustrated in Fig. 12, in a real implant treatment, using the auxiliary instrument 200 and the hole formation instrument 40, in the inferior alveolar bone 312 and the gum 316, the concave hole 318 is formed. The concave hole 318 has a depth obtained by adding up a distance D1 from a top portion of the inferior alveolar bone 312 to a bottom portion of the concave hole 318 and a distance (offset value) D2 from a top portion (defining surface) 335 of the inferior alveolar bone 312 to the defining surface 220 of the auxiliary instrument 200. In other words, when the auxiliary instrument 200 is used, the depth of the concave hole 318 is offset from the top portion of the inferior alveolar bone 312 to the defining surface 220 of the auxiliary instrument 200.

In this case, as illustrated in Figs. 12 and 13, the dentist selects each of the hole formation instrument 40 and the implant body 30 such that (Length H1 of Drill Body 42 below Upper End 46b of Drill Sleeve 46) - (Height H2 of Stopper 48) = (Length D1 of Implant Body 30 to be Used During Operation) + (Offset Value D2 When Auxiliary Instrument 200 is Used) is satisfied. The dentist forms the concave hole 318 such that a bottom portion of the concave hole 318 or a bottom portion of the implant body 30 is spaced apart from the not-to-be-reached target 320, and embeds the implant body 30 in the concave hole 318. In other words, the dentist uses the shape (e.g., outer diameter), the position, and the angle of the implant body 30 or an inner diameter, a position, and an angle of the concave hole 70 as parameters, while inputting the parameters H1, H2, D1, and D2 described above by appropriately using the operation unit 20, and creates an optimum treatment plan, while checking a state with respect to the treatment target area of the patient. Setting or installation of the parameter H1 and H2 includes selection of the optimum hole formation instrument 40 by the dentist.

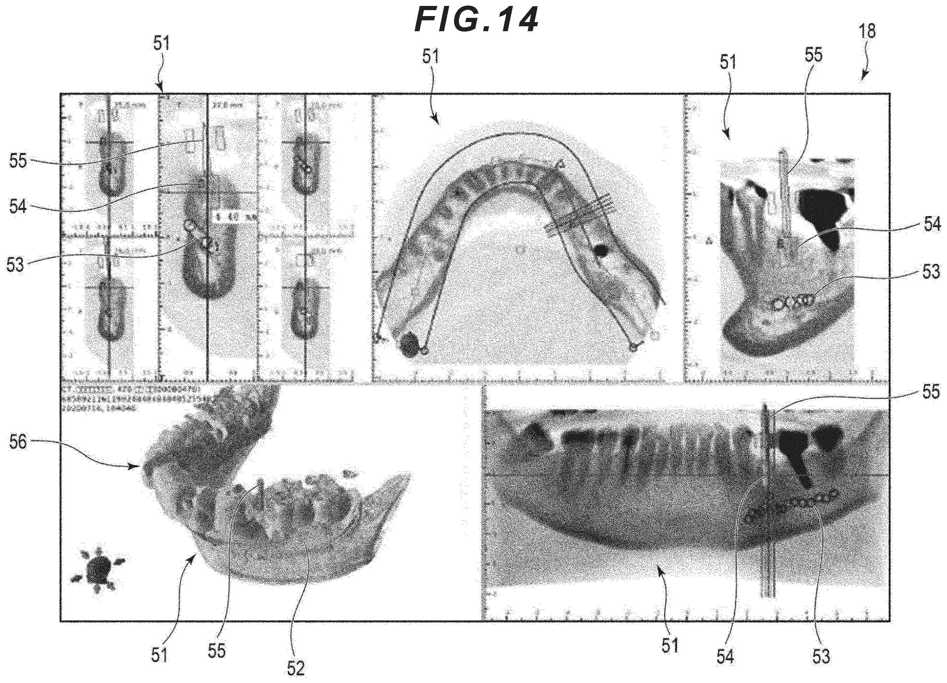

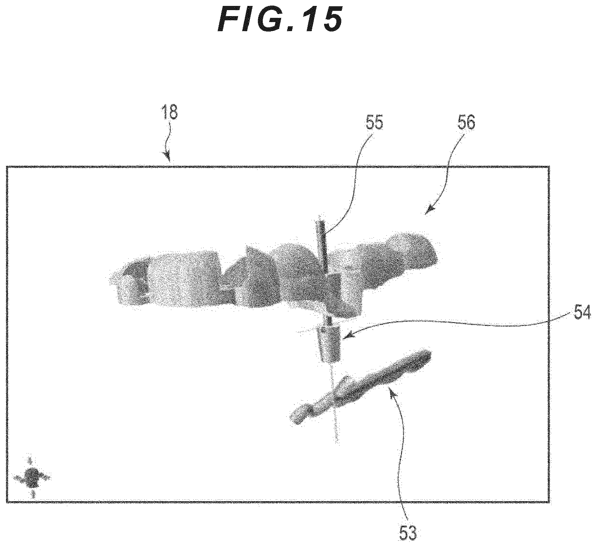

As illustrated in Fig. 14, the dentist superposes the first three-dimensional image 51 of the bone in the low jaw, the second three-dimensional image 52 of the teeth and the gum surface, the third three-dimensional image 53 of the not-to-be-reached target, the fourth three-dimensional image 54 of the implant body and/or the fifth three-dimensional image 55 of the hole formation instrument, and the sixth three-dimensional image 56 of the auxiliary instrument in a predetermined coordinate system (Step S7 or Fifth Processing). Then, as illustrated in Fig. 15, the dentist checks, on the display unit 18, a state in which the sixth three-dimensional image 56, the fourth three-dimensional image 54 or the fifth three-dimensional image 55, and the third three-dimensional image 53 are arranged. In other words, the dentist clearly shows, in the software, positional relationships between the auxiliary instrument, the implant body, the hole formation instrument that forms a hole in which the implant body is to be embedded, and the not-to-be-reached target.

Note that Steps S3 to S7 are processed using an image processing program, and the display unit 18 is caused to perform display using the image display program.

Then, the dentist forms the auxiliary instrument 200 with the 3D printer 24 or the milling machine 26 (Step S8).

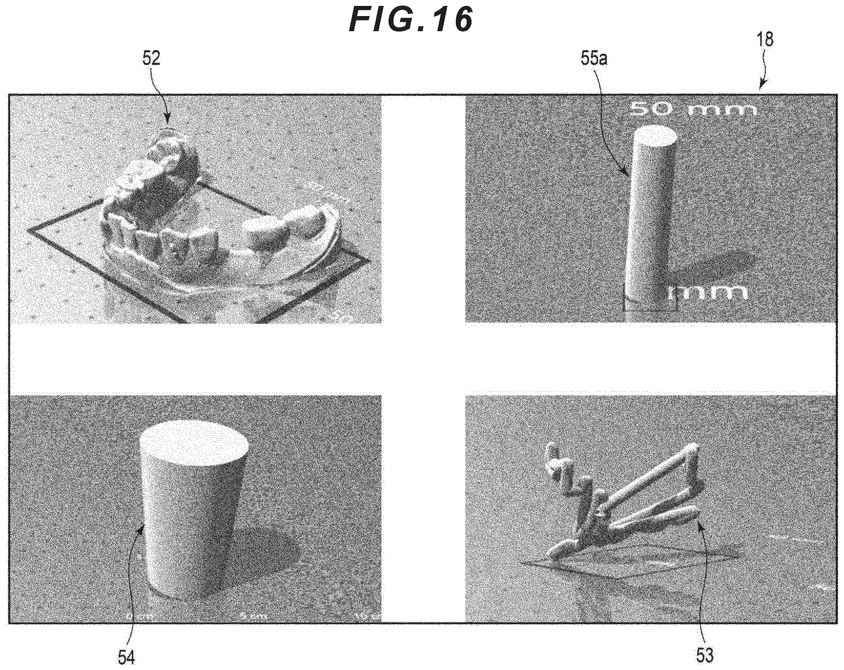

The dentist checks whether or not the treatment plan in the software has a point to be corrected. When there is a problem, a correction is made. When there is no problem, the second three-dimensional image data 52, the third three-dimensional image data 53, the fourth three-dimensional image data 54, and the fifth three-dimensional image data 55 each illustrated in Fig. 16 is superposed in the predetermined coordinate system (Step S9). At this time, using the software that creates the treatment plan, the second three-dimensional image data 52, the third three-dimensional image data 53, the fourth three-dimensional image data 54, and the fifth three-dimensional image data 55 is superposed in the predetermined coordinate system. Otherwise, when the data has compatibility, the dentist may also import these three-dimensional image data sets into, e.g., 3D CAD software other than the software that creates the treatment plan, and superpose these three-dimensional image data sets. At this time, as the fifth three-dimensional image data 55 of the hole formation instrument, e.g., only an image of the sleeve (guide tube) (fifth three-dimensional image data 55a related to the sleeve) except those of the body and the shank is imported.

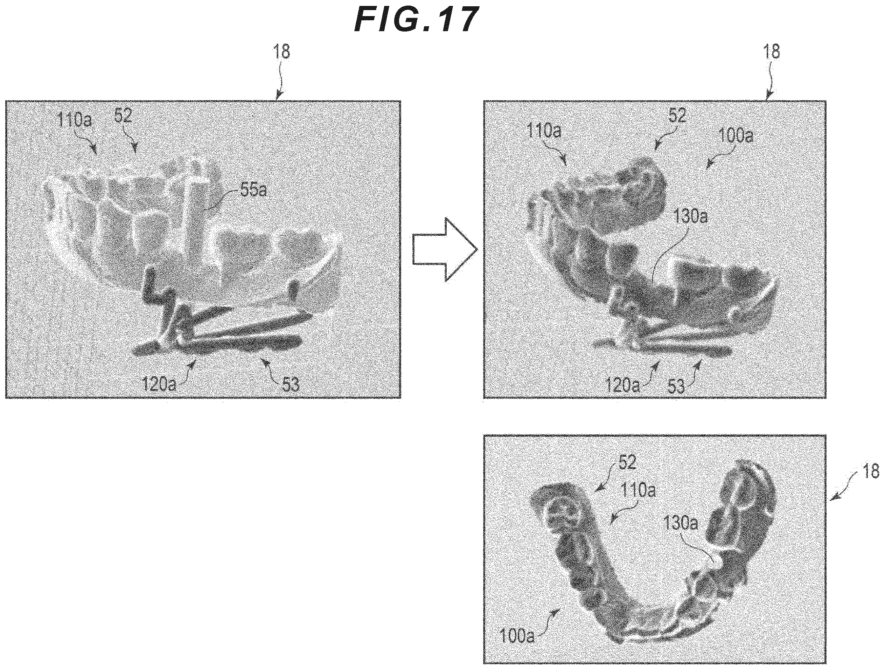

As illustrated in Fig. 17, the dentist removes, in the software, the fourth three-dimensional image data 54 related to the implant body and the fifth three-dimensional image data 55a related to the sleeve (Step S10 or Third Processing). In other words, in the software, three-dimensional image data (fifth surface data) 100a of the model 100 including three-dimensional image data 110a of the model main body 110 having the teeth and the through hole 130 imitating the concave hole in which the implant body is to be embedded and three-dimensional image data 120a of the target model portion 120 for the 3D printer 24 is produced.

Steps S9 to S10 are processed using the image processing program, and the display unit 18 is caused to perform display using the image display program.

After the dentist checks the data for the 3D printer 24, according to an operation input instruction to the operation unit 20 from the dentist, the 3D printer 24 controlled by the control device 12 forms the model 100 (see Fig. 4A and Fig. 4B) of the treatment area of the patient including the target model portion 120 imitating the teeth, the gum having the through hole 130 imitating the concave hole 70 in which the implant body 30 is to be embedded, and the inferior alveolar artery (Step S11 or fourth processing).

The dentist temporarily ends the processing of creating the treatment plan using the system 10 herein.

Thus, to the operation unit 20 of the system 10 described above, a processing instruction to specify, in the three-dimensional image data of the patient, the treatment target area of the patient and the not-to-be-reached target and a processing instruction to set, in the three-dimensional image data of the patient, various parameters of the implant body to be embedded in the treatment target area or install data acquired by the dentist by using the 3D scanner or the like and set a hole imitating the concave hole, in which the implant body is to be embedded, are given to the control device 12. In addition, to the operation unit 20, a processing instruction (coordinate conversion instruction) to superpose the first surface data (data related to a surface image), the second surface data or third surface data, and fourth surface data in the predetermined coordinate system and a processing instruction to subtract the second surface data and the third surface data from the first surface data to produce the fifth surface data, representing a positional relationship between the treatment target area including the hole and the fourth surface data, are input to be given to the control device 12.

In other words, to the operation unit 20, the processing instruction (coordinate conversion instruction) to superpose the first surface data, at least one of the second surface data and the third surface data, and the fourth surface data in the predetermined coordinate system and the processing instruction to subtract the second surface data and the third surface data from the first surface data and produce the fifth surface data, representing the positional relationship between the treatment target area including the hole and the fourth surface data, are input to be given to the control device 12, and the control device 12 processes these. Note that, when the second surface data is not used in the coordinate conversion instruction, the second surface data need not be subtracted during the production of the fifth surface data. When the third surface data is not used in the coordinate conversion instruction, the third surface data need not be subtracted during the production of the fifth surface data.

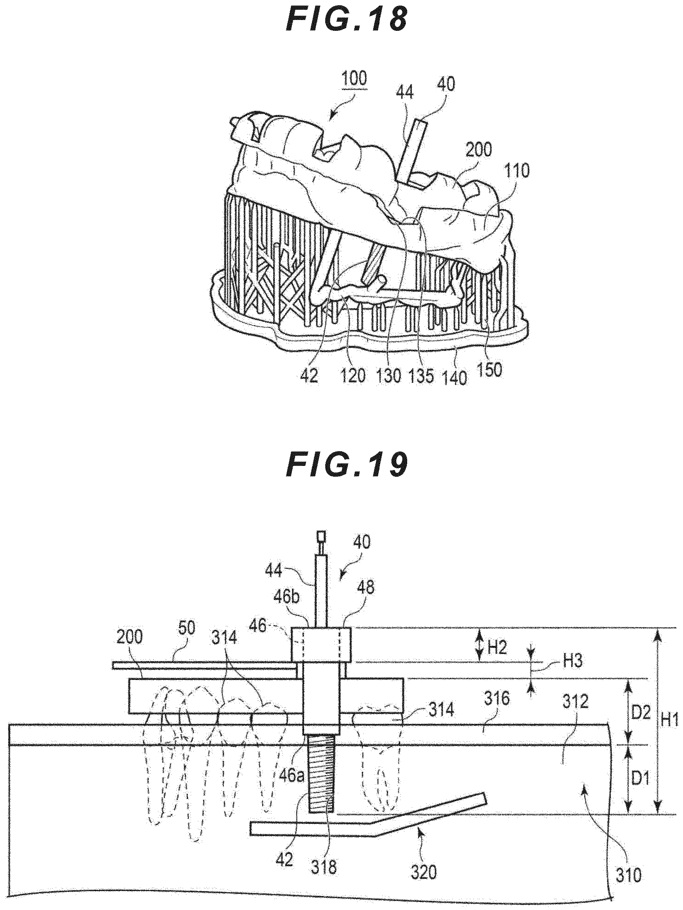

As illustrated in Fig. 18, the dentist fits, e.g., the auxiliary instrument 200 to the model 100 formed with the 3D printer 24. The dentist further inserts the body 42 of the hole formation instrument 40 into the guide hole 210 of the auxiliary instrument 200. At this time, the hole formation instrument 40 uses the sleeve 46 and the stopper 48. The dentist visually recognizes a positional relationship between the distal end of the body 42 of the hole formation instrument 40 and the target model portion 120. Specifically, when fitting the hole formation instrument 40 into the guide hole 210 of the auxiliary instrument 200 and inserting the body 42 of the hole formation instrument 40 into the through hole 130 in order to open the concave hole in which the implant body 30 is to be embedded, the dentist checks whether or not the body 42 of the hole formation instrument 40 is oriented in an intended direction and whether or not the distal end of the body 42 maintains a state spaced apart from the target model portion 120 of the artery, the nerve, or the like.

Alternatively, it is recommended that the distal end of the hole formation instrument 40 is 3 mm or more spaced apart from each of the inferior alveolar nerve and the inferior alveolar artery. Accordingly, it is also possible to create a treatment plan such that the target model portion 120 of each of the inferior alveolar nerve and the inferior alveolar artery is produced to be, e.g., 3 mm or more larger than the real inferior alveolar nerve or inferior alveolar artery toward the defining surface 135 and prevent the distal end of the hole formation instrument 40 from coming into contact therewith and being separated therefrom. In other words, by bringing a portion of the target model portion 120 closer to the defining surface 135 side than a position of the real not-to-be-reached target, the dentist can determine whether or not the hole formation instrument 40 to be actually used for the patient is usable on the basis of a positional relationship between a position at which the distal end of the body 42 of the hole formation instrument 40 came into contact with the target model portion 120 and the end surface 48a of the stopper 48 of the hole formation instrument 40 on the body 42 side. In other words, the dentist can also form the target model portion 120 as, e.g., a to-be-reached (to-be-contacted) target.

By thus using the system 10 and using the three-dimensional images 51 and 52 of the patient and the three-dimensional images 54 and 55 of the implant body and the hole formation instrument, the dentist forms the model 100 having the same size and shape as those of the real patient and can thereby perform pre-verification of a procedure of forming the concave hole in which the implant body 30 is to be embedded by using the hole formation instrument 40 to be actually used. In the pre-verification, when there is no problem, the dentist actually performs the procedure on the real patient exactly according to the treatment plan. When a problem arises in the pre-verification, the dentist corrects the treatment plan as necessary, produces the model 100 and the auxiliary instrument 200 again, and performs the pre-verification of the procedure. As necessary, the dentist repeats this task until there is no problem in the pre-verification.

Note that, when the model 100 is formed, the dentist may also selectively use the three-dimensional images 54 and 55 of the implant body and the hole formation instrument.

The through hole 130 of the model main body 110 is formed to have the position, the size, the angle, and the like thereof each having a final size that allows the implant body 30 to be fitted therein. In the real procedure, the dentist gradually increases a hole diameter from that of a small hole and deeply cuts down the hole. Accordingly, when forming the concave hole in the real procedure, the dentist advances the procedure, while gradually changing the short drill body 42 with a small diameter to the thicker drill body 42 with a larger diameter. In the system 10, it is possible to describe, as the treatment plan, what hole formation instrument the dentist uses to form the concave hole but, to the three-dimensional image formed with the 3D printer 24, the concave hole having a final size that allows the implant body 30 to be embedded therein can be set.

By using the model 100, the dentist can even perform pre-verification of advancement of the procedure, while gradually changing the short drill with the small diameter to the longer drill with the larger diameter. In other words, by using the model 100, the dentist can perform pre-verification not only with the hole formation instrument 40 to be finally used, but also with various procedure instruments to be used during formation of the concave hole. Examples of the procedure instruments to be used during the formation of the concave hole include an injector that injects, into the treatment target area, a bone prosthetic material, a fibrin gel including a blood platelet separated from collected blood, or a mixture thereof, and the like.

At present, a dentist may calculate, during a real procedure, various parameters such as a length of the body 42 of the hole formation instrument, a height of the stopper 48, and an offset value according to the implant body 30 to be used in the procedure, and perform the procedure. When the dentist makes a mistake with any one of the parameters, an instrument different from an instrument to be originally used may be used, which may lead to a medical accident. By using the model 100 according to the present embodiment, the dentist can personally perform pre-validation of adequacy of the use of each procedure instrument until the formation of the concave hole with the final size in the real procedure.

Meanwhile, a creator of the treatment plan in, e.g., the system 10, such as a dentist, may make a mistake with various parameters such as the length of the body 42 of the hole formation instrument 40, the height of the stopper 48, and the offset value during the creation of the treatment plan. Then, the creator of the treatment plan may overlook errors in the various parameters and end the creation of the treatment plan. In this case also, when performing pre-verification of the treatment plan by using the model 100 and the real hole formation instrument 40, the dentist can notice mistakes in the setting of the various parameters in the treatment plan. Then, the dentist can examine, using the model 100, how parameters such as the length of the body 42 of the hole formation instrument 40 to be used, the height of the stopper 48, and the offset value are to be changed to successfully perform the procedure. At this time, a hole formation instrument other than the hole formation instrument 40 from the manufacturer set during the creation of the treatment plan can also be tried appropriately. Accordingly, by using the model 100, the dentist can appropriately select the hole formation instrument 40 from among instruments owned by the dentist.

Therefore, the pre-verification of the procedure performed by the dentist by using the model 100 described in the present embodiment should inevitably be performed as a part of treatment in the implant treatment. By thus performing the pre-verification of the procedure using the model 100, the dentist can appropriately change the hole formation instrument 40 as necessary, and perform an optimum treatment. Needless to say, the dentist can correct the treatment plan by using the system 10. Accordingly, the use of the model 100 according to the present embodiment by the dentist can significantly increase the safety of an implant treatment.

In the implant treatment, e.g., the auxiliary instrument 200 is produced, and the dentist performs a treatment that forms a concave hole of a predetermined size at an intended position in the lower jaw of the patient and embeds the implant body 30 in the concave hole. Conventionally, due to a relationship between the hole formation instrument 40 for embedding the implant body 30 and the auxiliary instrument 200, there is no means for the dentist to actually visually check whether or not, e.g., 42 of the hole formation instrument 40 from another manufacturer based on a unique idea, which is selected as an alternative, reaches the not-to-be-reached target. According to the present embodiment, the relationships between the model main body 110 including the treatment target area of the patient, the target model portion 120, the auxiliary instrument 200, and the hole formation instrument 40 or the implant body 30 can be verified by the dentist by using the model 100 and the real hole formation instrument 40 or the implant body 30 before the procedure. In other words, the dentist can perform pre-verification of the created treatment plan before the real procedure. Therefore, the dentist can perform the real procedure after performing pre-verification of safety of the procedure by using the hole formation instrument 40. Since the dentist has preliminarily recognized, in the model 100, a position reached by the distal end of the body 42 of the hole formation instrument 40 with respect to the not-to-be-reached target, i.e., a separation distance between the not-to-be-reached target and the distal end of the body 42 of the hole formation instrument 40 or a contact state during a real procedure, it is possible to further shorten time taken by the dentist to perform the procedure. Accordingly, the dentist can perform a less invasive procedure on the patient by performing the procedure exactly according to the treatment plan.

**The** dentist can perform processing of creating the treatment plan by using the system 10 between medical practice that acquires patient data using the first scanner (CT scan) 14, the second scanner (intraoral scanner) 16 and medical practice that actually performs a procedure on the patient. In the present embodiment, the description has been given of an example in which the dentist personally creates the treatment plan. The processing of creating the treatment plan is extremely important processing which does not actually treat or diagnose the patient, but leads to medical practice which is a procedure of embedding the implant body 30 in the lower jaw. Accordingly, creation of the treatment plan using the system 10 and pre-verification of the procedure using the model 100 produced on the basis of the treatment plan as well as the auxiliary instrument 200, each performed by the dentist, becomes extremely effective processing in ensuring safety of the implant treatment. For this purpose, it is extremely useful for the dentist to use the system 10 capable of creating a treatment plan optimal for each patient.

As described above, it may be possible that, e.g., an unqualified person for dental therapeutic practice, such as a manufacturer technician or a dental technician, creates the treatment plan. In this case also, the dentist can receive treatment plan creation data, examine the treatment plan in the system 10, and personally give a correction instruction or correct the treatment plan. In either case, the dentist can previously verify safety of the treatment by using the model 100, the hole formation instrument 40, the implant body 30, and the auxiliary instrument 200 immediately before the actual procedure is performed.

When correcting the treatment plan described above and re-producing the model 100, the dentist can reduce time required to communicate with a supplier such as a manufacturer by personally performing a sequence of tasks by using the system 10. As a result, when the dentist produces the treatment plan and outputs the model 100 with the 3D printer 24, it is possible to achieve a significant time reduction, such as, e.g., on a per week basis, compared to that when the dentist uses the supplier. Therefore, the dentist can establish a state where the procedure can be performed earlier on the patient.

When the dentist uses the system 10, the dentist can take the lead in developing an optimal treatment plan through a trial and error process. As a result, even if the output of the auxiliary instrument 200 and the model 100 is entrusted to the supplier, it is possible to reduce the number of times the auxiliary instrument 200 and the model 100 are corrected. Therefore, the dentist can establish a state where the procedure can be performed earlier on the patient.

Note that, conventionally, the cost of producing an auxiliary instrument (surgical guide) is relatively high, for example, at least 35,000 yen or more and, since the auxiliary instrument is not necessarily required, it may be difficult to persuade a patient to pay for the cost of producing the auxiliary instrument 200, even when a dentist uses the auxiliary instrument 200. For this reason, it is difficult to say that the use of an auxiliary instrument is widespread in a current implant treatment. However, by personally designing the auxiliary instrument 200 by using the system 10 according to the present embodiment and outputting the auxiliary instrument 200 by using the 3D printer 24 or the milling machine 26 owned by the dentist, the dentist can achieve a significant cost reduction in producing the auxiliary instrument 200. Therefore, by using the system 10 according to the present embodiment, it is possible to promote the use of an auxiliary instrument such as a surgical guide by a dentist during an implant treatment.

Accordingly, by using the system 10, it is possible to change the creation of the treatment plan to the initiative of the dentist as much as possible, reduce the cost of creating the treatment plan including the auxiliary instrument 200, and spread a safer treatment using the auxiliary instrument 200.

As has been described heretofore, according to the present embodiment, it is possible to provide the pre-procedural verification system 10 for a dental treatment plan, a pre-procedural verification program for a dental treatment plan, a method of producing the model 100 for pre-procedural verification of a dental treatment plan, and the model 100 for pre-procedural verification of a dental treatment plan which allow, when, e.g., a treatment that embeds an embedded body such as, e.g., an implant body is to be performed, a dentist to preliminarily verify, before a real dental treatment using a procedure instrument such as the hole formation instrument 40, a positional relationship between a distal end position of the procedure instrument and a target position such as a position of an inferior alveolar artery or an inferior alveolar nerve in a lower jaw of a patient when the procedure instrument is used.

In the model 100 illustrated in Figs. 4A and 4B, a portion corresponding to the inferior alveolar bone of the patient is not present. As illustrated in Fig. 4C, the model 100 produced with the 3D printer 24 may also have, as a membranous body 145, a portion corresponding to the inferior alveolar bone in which the resin material of the model 100 is transparent or translucent to allow the dentist to visually recognize the target model portion 120. When, e.g., the resin material of the model 100 is transparent or translucent, the model 100 is formed into a state where the fourth three-dimensional image 54 of the implant body and the fifth three-dimensional image 55 of the hole formation instrument have been removed from a lower left view in Fig. 10. The portion corresponding to the inferior alveolar bone can also be formed into a mesh-like structure as long as the positional relationship between the hole formation instrument 40 and the target model portion 120 is visually recognizable.

The auxiliary instrument 200 has the guide hole 210 for forming the concave hole in which the implant body 30 is to be embedded. The guide hole 210 and the concave hole 70 are circular holes when the guide hole 210 and the concave hole 70 are formed with a rotary drill as the hole formation instrument 40. As the guide hole 210 or the concave hole 70, a non-circular hole may also be formed without using the rotary drill depending on the hole formation instrument 40.

In the present embodiment, the description has been given of the example in which the two scanners, which are the first scanner 14 and the second scanner 16, are used. For example, as long as the first three-dimensional image data 51 and the second three-dimensional image data 52 can be acquired with one scanner, a plurality of scanners may be unnecessary.

The first scanner 14 and the second scanner 16 are owned by the dentist, and the dentist acquires three-dimensional image data of the jaw region of the patient. The formation of an object with the 3D printer 24 may also be performed by an appropriate supplier. For example, it may be possible to preliminarily acquire three-dimensional image data of the patient by using the first scanner 14 and/or the second scanner 16, and accordingly the first scanner 14 and/or the second scanner 16 is also preferably not included in the system 10. The 3D printer 24 and/or the milling machine 26 is also preferably not included in the system 10.

In the present embodiment, the description has been given of the example in which the auxiliary instrument 200 is produced in the system 10, and the auxiliary instrument 200 is used for the real procedure. The auxiliary instrument 200 may not be necessarily required depending on, e.g., the selection of the hole formation instrument 40.

### (First Modification)

Using Fig. 19, a description will be given of the system 10 according to the first modification of the first embodiment. A description will be given herein of differences in parameter setting when the hole formation instrument 40 different from the hole formation instrument 40 illustrated in Fig. 3 is used.

Fig. 19 illustrates the first modification of the hole formation instrument 40 different from that in Fig. 3. The hole formation instrument 40 illustrated in Fig. 19 has a handle 50 in addition to the body 42, the shank 44, the sleeve 46, and the stopper 48. A lower end of the stopper 48 comes into contact with the handle 50. Accordingly, the position of the distal end of the body 42 of the hole formation instrument 40 can be adjusted by a height of the handle 50. For example, when the height of the handle 50 is large, a bottom portion of the concave hole 318 is away from the not-to-be-reached target 320.

As illustrated in Fig. 19, the dentist selects each of the hole formation instrument 40 and the implant body 30 such that (Length D1 of Implant Body 30) + (Offset Value D2 When Auxiliary Instrument 200 is Used) = (Length H1 of Drill Body 42 below Upper End of Drill Sleeve 46) - (Height H2 of Stopper 48) - (Height H3 of Handle 50). Thus, the dentist sets various set values according to the various hole formation instruments 40.

In other words, the dentist uses, as parameters, the shape (length or outer diameter), the position, and the angle of the implant body 30 and also appropriately inputs the parameters H1, H2, H3, D1, and D2 described above by using the operation unit 20 to create the optimum treatment plan, while checking a state with respect to the treatment target area of the patient. The setting or installation of the parameters H1, H2, and H3 includes selection of the optimum hole formation instrument 40 by the dentist.

### (Second Modification)

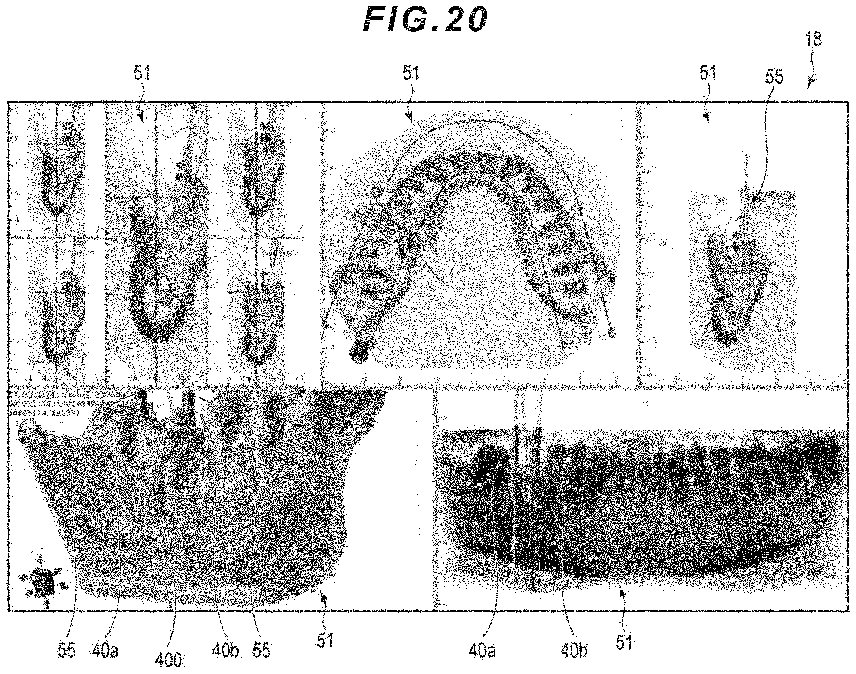

In the first embodiment, as illustrated in Fig. 12, the description has been given of the case where the implant body 30 is embedded in the concave hole 318 formed in the lower jaw. In a case of forming, e.g., a concave hole in which an autologous tooth 400 is to be embedded as an embedded body illustrated in Fig. 20 instead of embedding the implant body 30 in the concave hole 318 also, the treatment plan can similarly be created by using the system 10 described in the first embodiment.

The dentist can produce three-dimensional image data (second surface data) of the autologous tooth 400 in the same manner as producing the three-dimensional image data of the implant body 30. The three-dimensional image data of the autologous tooth 400 can be acquired by using various devices. The three-dimensional image data of the autologous tooth 400 may also be acquired using, e.g., the second scanner 16.

The dentist uses a shape, a position, and an angle of the autologous tooth 400 as parameters and also inputs the parameters H1, H2, H3, D1, and D2 described above by appropriately using the operation unit 20 to create the optimum treatment plan, while checking a state with respect to the treatment target area of the patient. The setting or installation of the parameters H1, H2, and H3 includes selection of the optimum hole formation instrument 40 by the dentist. Unlike the implant body 30 for which a ready-made product can be used, the autologous tooth 400 has a shape differing from one patient to another. Accordingly, when the autologous tooth 400 is to be embedded in the lower jaw, two or three concave holes may be opened according to the shape of the autologous tooth 400. Even when these concave holes are formed also, the auxiliary instrument 200 can be used. In the case of forming the concave holes, e.g., a plurality of hole formation instruments 40a and 40b which are the same or different can be used in succession.

Note that when regenerative medicine using, e.g., a stem cell of a tooth instead of the autologous tooth is to be practiced, it may be possible to perform a treatment that embeds, in the concave hole, the stem cell of the tooth cultured in vivo or in vitro. In this case, in the creation of a treatment plan in the system 10, the dentist uses a shape, a position, and an angle of a cell tissue (e.g., an aggregate of cell tissues) as the embedded body in regenerative medicine as parameters, and also inputs the parameters H1, H2, H3, D1, and D2 described above by appropriately using the operation unit 20 to create the optimum treatment plan, while checking a state with respect to the treatment target area of the patient. The setting or installation of the parameters H1, H2, and H3 includes selection of the optimum hole formation instrument 40 by the dentist. Thus, even when practicing the regenerative medicine by using the system 10 described above, the dentist can create the treatment plan and produce a model for pre-procedural verification of the dental treatment plan.

### (Second Embodiment)

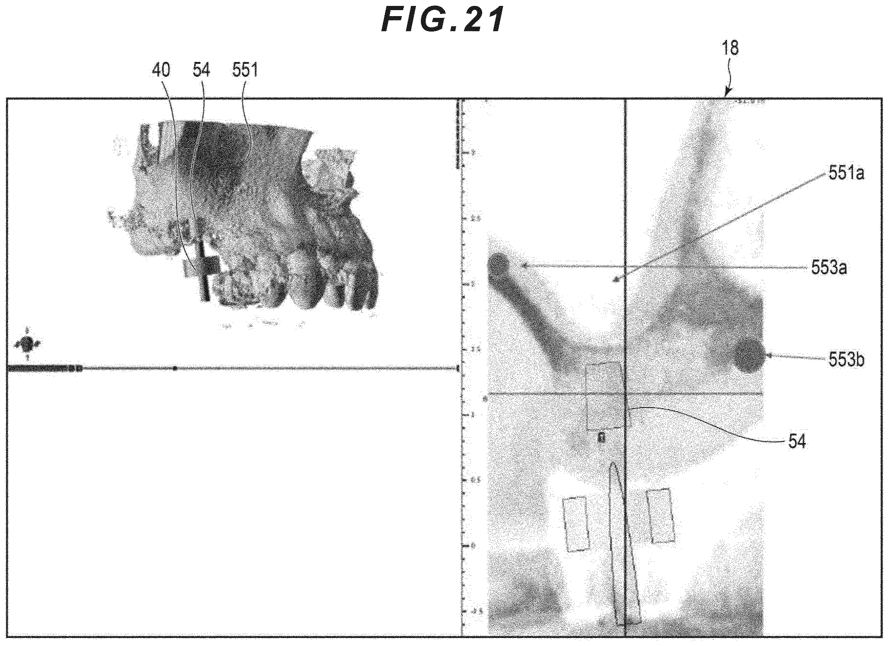

In the second embodiment, using Figs. 21 to 29, a description will be given of a case where the system 10 is used for an example in which an implant treatment that embeds the implant body 30 (see Fig. 2) selected from among a large number of implant bodies in an upper jaw of a patient is performed. A description of matters common to the matters described in the first embodiment is omitted as appropriate.

The first scanner (dental CT scan) 14 acquires first three-dimensional image data (e.g., DICOM data) 551 of, e.g., teeth, a bone, and the inside of the bone in the upper jaw of the patient, and outputs the first three-dimensional image data 551 to the control device 12. The control device 12 causes the storage device 22 to store the first three-dimensional image data 551. The second scanner (intraoral scanner) 16 acquires second three-dimensional image data (e.g., STL data) 552 of, e.g., teeth and a gum surface in the upper jaw of the patient and outputs the second three-dimensional image data 552 to the control device 12. The control device 12 causes the storage device 22 to store the second three-dimensional image data 552.

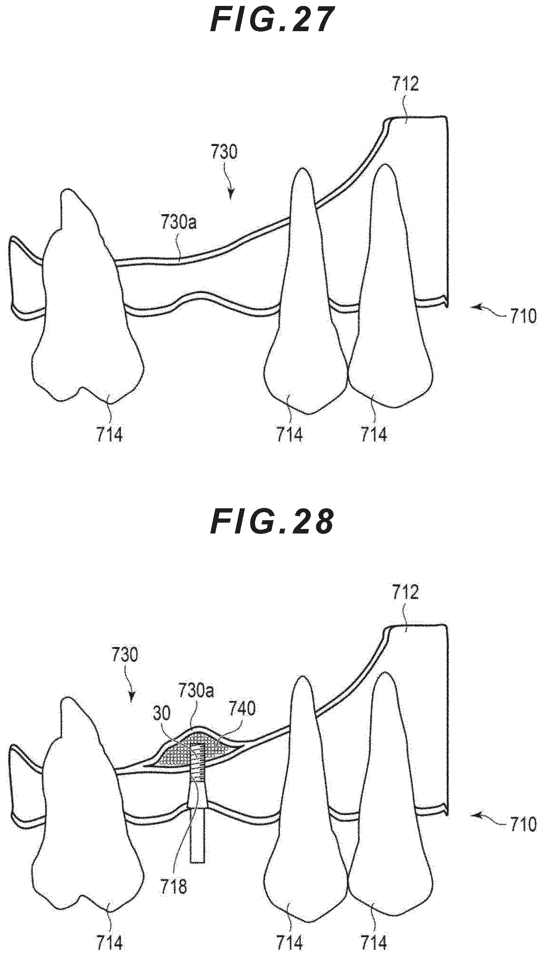

As illustrated in Fig. 21, the dentist uses, in software, the first three-dimensional image 551 representing a superior alveolar bone, a maxillary sinus, a posterior superior alveolar artery, and a greater palatine artery, which is obtained by the first scanner 14, to specify positions of the posterior superior alveolar artery, the greater palatine artery, and a maxillary sinus floor mucosa (see Figs. 27 to 29) that are to be used as the not-to-be-reached targets during setting of the concave hole 70 in which the implant body is to be embedded. Then, the dentist marks feature points of the not-to-be-reached targets in the software such that the not-to-be-reached targets are three-dimensionally formed (Step S3). Then, the dentist produces, as third three-dimensional image data 553a, 553b, and 553c, the not-to-be-reached targets. Note that the posterior superior alveolar artery, the greater palatine artery, and the maxillary sinus floor mucosa are adjacent to a superior alveolar bone.

A right view in Fig. 21 illustrates the three-dimensional image 553a of a an area of the maxillary sinus 551a and an area of the posterior superior alveolar artery and the three-dimensional image 553b of an area of the greater palatine artery.

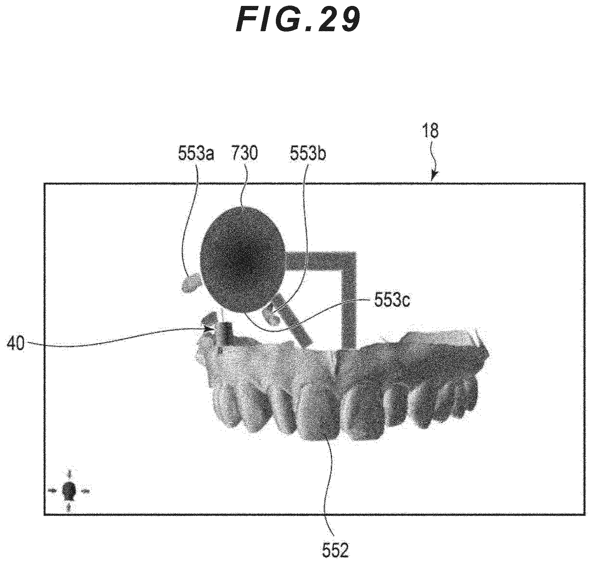

Note that the three-dimensional image data 553c corresponding to the maxillary sinus floor mucosa may be produced as a portion of an eggshell or may also be formed into, e.g., a spherical shape (see Fig. 29) as a whole. This is because, when pre-verification of the treatment plan is performed using the model, an area that affects reach of the distal end of the body 42 of the hole formation instrument 40 corresponds to the maxillary sinus floor mucosa, and the remaining area does not affect the reach of the distal end of the body 42 of the hole formation instrument 40.

As illustrated in Fig. 21, the dentist sets or installs, in the software, a length, an outer diameter, an angle, a position, and the like of an implant body imitating the implant body 30 at a treatment position (Step S4). At this time, the dentist appropriately sets the outer diameter, position, angle, and various parameters (including the selection of the hole formation instrument 40) of the implant body 30 described in the first embodiment to optimize the treatment plan.

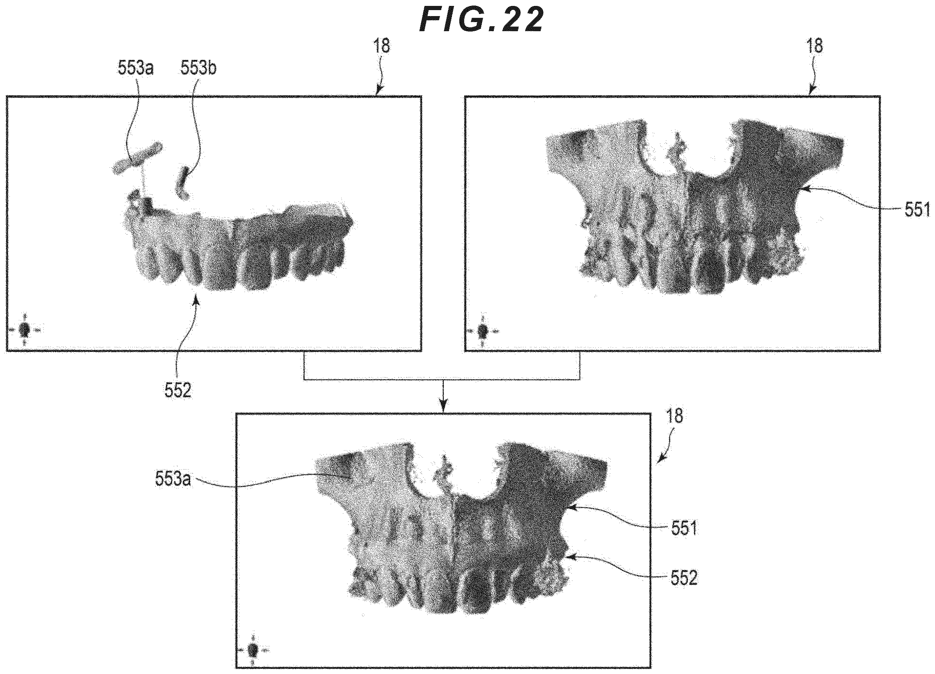

As illustrated in Fig. 22, the dentist aligns, in the software, respective coordinate axes of the first to fifth three-dimensional image data 551, 552, 553a to 553c, 54, and 55 and superimposes the second three-dimensional image data 552 on the first three-dimensional image data 551, the third three-dimensional image data 553a to 553c, the fourth three-dimensional image data 54 and/or the fifth three-dimensional image data 55 in a predetermined coordinate system (Step S5). Accordingly, the dentist shows, in the software, a positional relationship between the teeth and the gum surface in the upper jaw and an artery inside the superior alveolar bone.

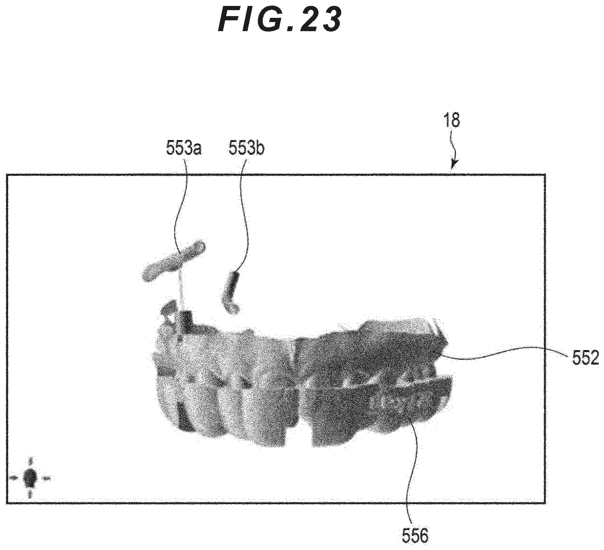

As illustrated in Fig. 23, the dentist produces, in the software, an auxiliary instrument (surgical template) according to the shape of the upper jaw of the patient (Step S6). In other words, as sixth three-dimensional image data 556, the dentist produces the auxiliary instrument. The dentist checks, on the display unit 18, a state in which the sixth three-dimensional image data 556, the fourth three-dimensional image data 54 or the fifth three-dimensional image data 55, and the third three-dimensional image data 553a and 553b is laid out (Step S7). Then, the dentist outputs the auxiliary instrument (Step S8).

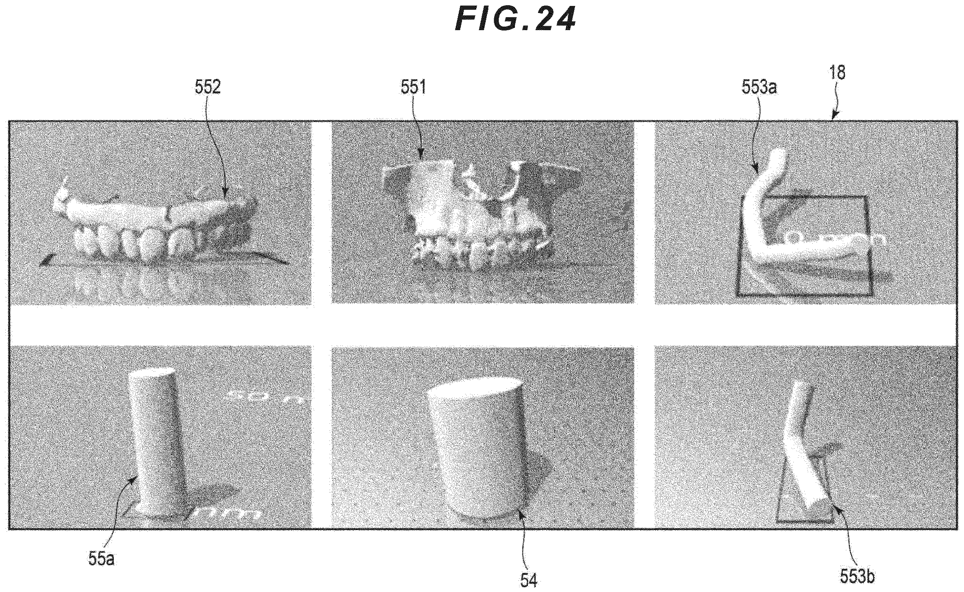

The dentist checks whether or not the treatment plan in the software has a point to be corrected. When there is a problem, a correction is made. When there is no problem, the first three-dimensional image data 551 representing the teeth and the superior alveolar bone, the second three-dimensional image data 552 representing the teeth and the gum, the third three-dimensional image data 553a and 553b of the not-to-be-reached targets, the fourth three-dimensional image data 54 of the implant body, and the fifth three-dimensional image data 55 of the hole formation instrument 40, which are illustrated in Fig. 24, are superposed in the predetermined coordinate system (Step S9). At this time, using the same software as the software used to create the treatment plan, the second three-dimensional image data 552, the third three-dimensional image data 553a and 553b, the fourth three-dimensional image data 54, and the fifth three-dimensional image data 55 are superposed in a predetermined coordinate system.

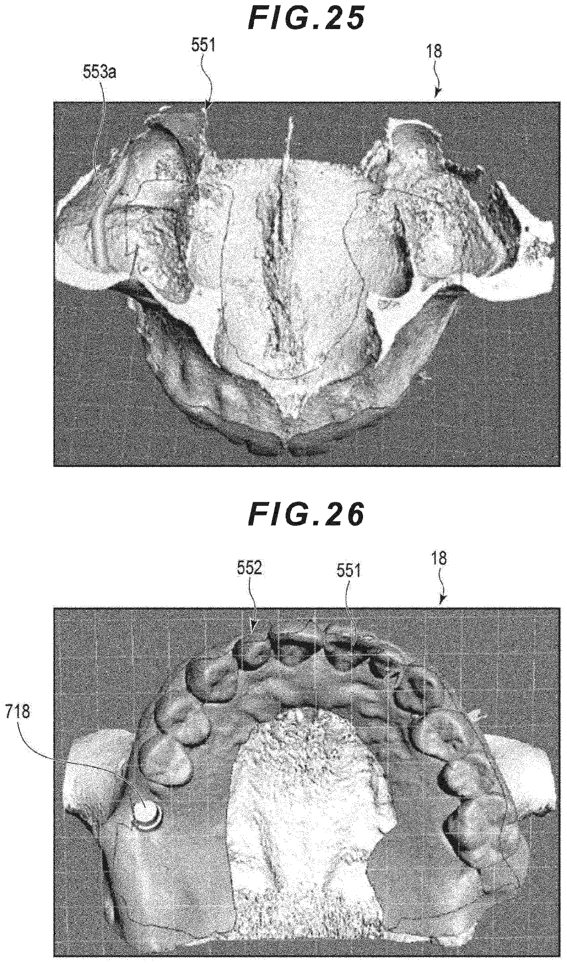

The dentist removes, in the software, the fourth three-dimensional image data 54 related to the implant body 30 and the fifth three-dimensional image data 55a related to the sleeve (Step S10). In other words, as illustrated in Figs. 25 and 26, data (surface data of the model) for the 3D printer 24 on the teeth, the gum having a through hole imitating the concave hole in which the implant body 30 is to be embedded, and the not-to-be-reached targets is produced in the software.

After the dentist checks the data for the 3D printer 24, the 3D printer 24 controlled by the control device 12 forms, on the basis of an instruction input by the dentist, a model of the treatment area of the patient which imitates the teeth, the gum having the through hole imitating the concave hole in which the implant body 30 is to be embedded, and the posterior superior alveolar artery and the greater palatine artery which correspond to the not-to-be-reached targets (Step S11).

Fig. 27 illustrates herein relationships between an alveolar bone 712, teeth 714, and a maxillary sinus 730 in an upper jaw 710. Fig. 28 illustrates a state where the implant body 30 is fixed to the upper jaw 710 using maxillary sinus floor elevation such as, e.g., a sinus lift (socket lift) procedure. When the implant body 30 is to be embedded in the superior alveolar bone 712 in the upper jaw 710, a thickness of the superior alveolar bone 712 illustrated in Fig. 27 may be insufficient. At this time, the maxillary sinus floor elevation that elevates a maxillary sinus floor mucosa 730a of the maxillary sinus 730 illustrated in Fig. 28 by using an artificial bone grafting material 740 is performed. In the case of performing the maxillary sinus floor elevation, the maxillary sinus floor mucosa 730a of the maxillary sinus 730 also serves as the not-to-be-reached target of the drill body 42 as the hole formation instrument 40 together with the posterior superior alveolar artery and the greater palatine artery.

When the maxillary sinus floor elevation is to be performed, a concave hole 718 in which the implant body 30 is to be embedded is opened in the superior alveolar bone 712. At this time, the concave hole 718 is opened to, e.g., the vicinity of a floor portion of the maxillary sinus 730, but the maxillary sinus floor mucosa 730a is not penetrated. The superior alveolar bone 712 is penetrated not with the hole formation instrument 40, but with an osteotome or the like. A space between the mucosa 730a of the maxillary sinus 730 and the superior alveolar bone 712 is filled with the bone grafting material 740. In this state, the implant body 30 is embedded. At this time, the bone grafting material 740 and the implant body 30 are kept from breaking the maxillary sinus floor mucosa 730a.

In this case, as a model for pre-procedural verification of a dental treatment plan, a model which is not formed with the superior alveolar bone, in the same manner that, e.g., the inferior alveolar bone described in the first embodiment is not formed, and which imitates teeth, a gum, a posterior superior alveolar artery, a greater palatine artery, and a maxillary sinus floor mucosa is formed as illustrated in Fig. 29. By thus forming the model, the dentist is allowed to check a positional relationship between the distal end of the body 42 of the hole formation instrument 40 and the maxillary sinus floor mucosa 730a when the selected hole formation instrument 40 is fitted into each of the guide hole of the auxiliary instrument and a through hole of the model. In other words, the dentist can perform pre-verification of the dental treatment plan to determine whether or not the dental treatment can more safely be performed.

Note that, when the areas corresponding to the teeth and the gum are used as a model main body, in the model of the upper jaw, e.g., the maxillary sinus floor mucosa 730a are preferably supported with braces, as in the model 100 described in the first embodiment.

In the same manner as when the system 10 is used for the lower jaw, even when the system 10 is used for the upper jaw also, the same effects as achieved on the lower jaw can be obtained.

As has been described heretofore, according to the present embodiment, it is possible to provide the pre-procedural verification system 10 for a dental treatment plan, a pre-procedural verification program for a dental treatment plan, a method of producing a model for pre-procedural verification of a dental treatment plan, and a model for pre-procedural verification of a dental treatment plan which allow, before a real dental treatment using a procedure instrument when, e.g., a treatment that embeds an embedded body, such as, e.g., an implant body, is to be performed, a dentist to preliminarily verify a positional relationship between a distal end position of the procedure instrument and a target position such as each of positions of a posterior superior alveolar artery, a greater palatine artery, and a maxillary sinus mucosa in an upper jaw of a patient when the procedure instrument is used.

Note that, in the system 10 according to the second embodiment also, it is possible to use the autologous tooth 400 or the cell tissue described in the modification of the first embodiment, instead of the implant body 30.

According to each of the first embodiment including the modification and the second embodiment each described above, before performing the treatment that embeds, e.g., the implant body 30 or the autologous tooth, the dentist can preliminarily verify, before the procedure, whether or not the treatment plan including the selection of the various treatment instruments has a problem by using the model 100 produced by using the system 10 for dental treatment pre-verification, the hole formation instrument (procedure instrument) 40 scheduled to be used in the real dental treatment, and the implant body 30 or the autologous tooth 400. Accordingly, it is possible to prevent such a situation in which the dentist becomes skeptical about whether or not he can perform, with a tool to be used during the procedure (for example, the hole formation tool 40), a predetermined treatment according to the treatment plan on the treatment target area of the patient, spends time on the treatment, and places burdens on the patient. Therefore, by using the system 10 and the model 100 according to the first embodiment including the modification and the second embodiment each described above and further using the hole formation instrument 40 set in the treatment plan, it is possible to perform a less invasive dental treatment on the patient.

**Additionally,** by allowing, not a supplier, but a dentist who actually performs a treatment to perform a task of creating the treatment plan thus using the system 10 until the three-dimensional image data of the model 100 is produced, while checking the task, it is possible to more safely perform the treatment that embeds, e.g., the implant body 30 or the autologous tooth.

Alternatively, when the dentist owns the 3D printer 24 or the milling machine 26, it is possible to eliminate time for delivery of the three-dimensional image data and transport time for the model 100, and reduce time spent on a sequential task from the creation of the treatment plan to the pre-verification of the dental treatment using the model 100. In addition, when the treatment plan is to be corrected also, it is possible to reduce time spent on a sequential task from the correction of the treatment plan to the pre-verification of the dental treatment using the model 100.

### (Third Embodiment)

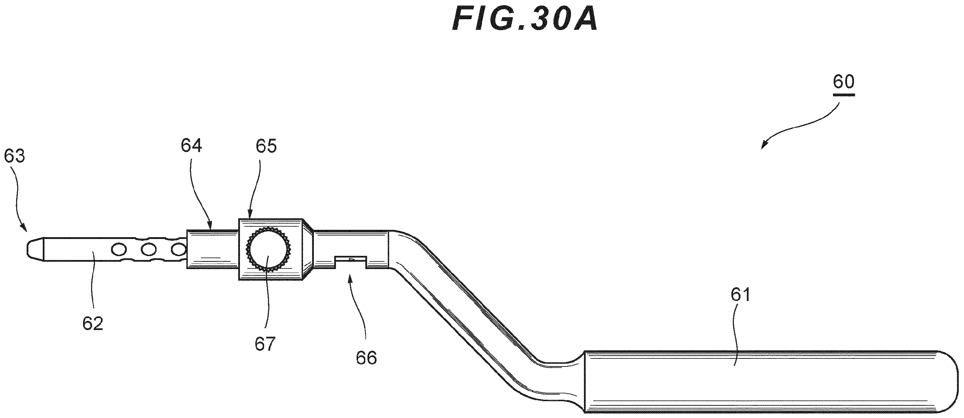

A third embodiment of the present invention relates to an osteotome for transplant. An osteotome 60 is used as an instrument which is embedded from a surface layer of a treatment area in a jaw region of a patient into a deep portion to apply an impact in expanding or compressing a bone so as to displace a position of a mucosa behind the treatment area. The osteotome 60 in the present embodiment includes a body portion 61, a bar portion 62, an end portion 63, a guided portion 64, a stopper portion 65, a display window 66, and a screw 67 (see Fig. 30A or the like).

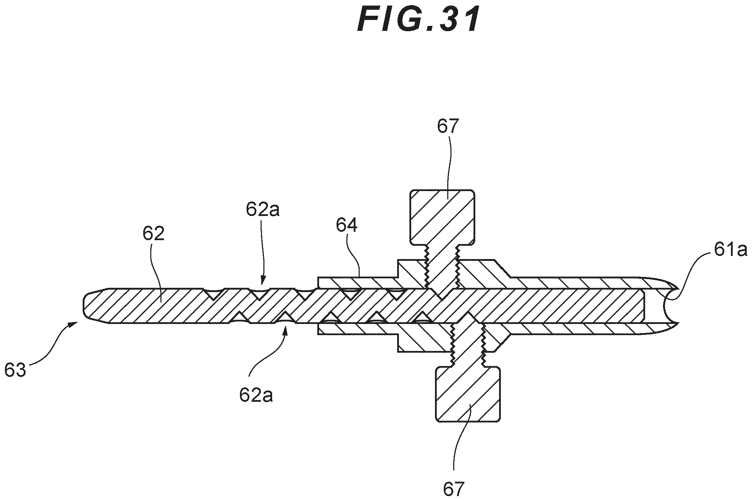

The body portion 61 is a main body portion of the osteotome 60 and has a shape that is easily held and operated by the dentist. The body portion 61 is formed with a hollow portion 61a for attachment of the bar portion 62 (see Fig. 31 or the like).

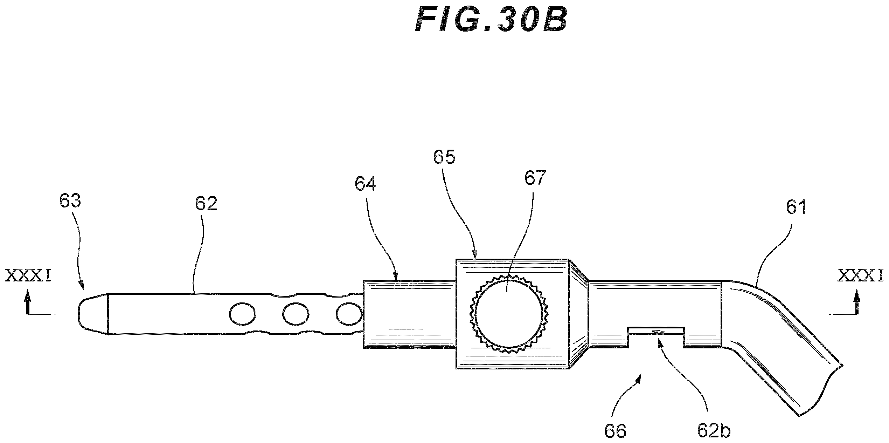

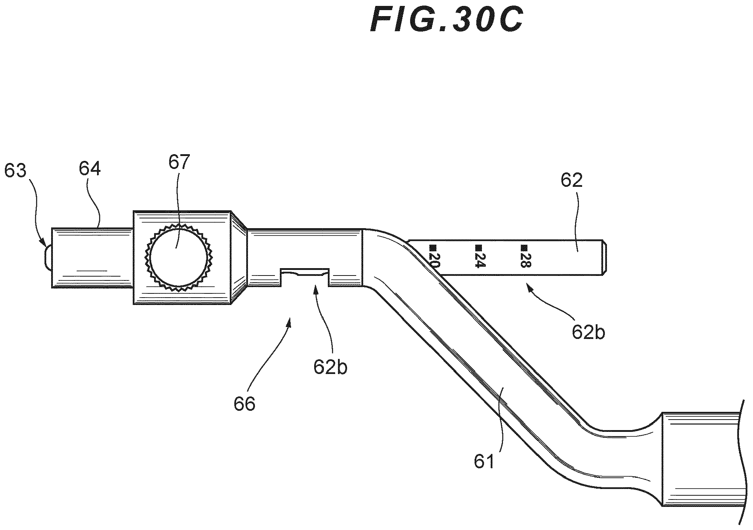

The bar portion 62 is a member having an elongated shape to be attached to the hollow portion 61a of the body portion 61 and having the end portion 63 formed on a distal end thereof so as to displace a position of a mucosa of the patient. The bar portion 62 in the present embodiment is formed to be slidable in a longitudinal direction along the hollow portion 61a and adapted to be able to freely change an amount of protrusion to the end portion 63 and an entire length of the osteotome 60 (see Figs. 30A, Fig. 30C, and the like). In addition, in the bar portion 62, a plurality of engagement grooves 62a are formed at predetermined intervals along the longitudinal direction (see Fig. 30B and the like). These engagement grooves 62a are formed such that a distal end of the screw 67 is engaged therewith when the screw 67 is rotated and, by thus engaging the distal end of the screw 67 with the engagement grooves 62a, it is possible to engage the bar portion 62 and determine a relative position of the bar portion 62 with respect to the body portion 61 (see Fig. 31). The bar portion 62 is also provided with an information presentation unit 62b that presents information related to the amount of protrusion to the end portion 63 in a state where positioning is performed by the screw 67 or information related to an embedment depth (see Fig. 30B and the like). For example, in the present embodiment, the information is represented by a numerical value (see Fig. 30B), but this is exemplary, and a specific method of information presentation by the information presentation unit 62b is not particularly limited.

The end portion 63 is a distal end portion of the bar portion 62, and is formed into a shape appropriate to come into contact with a mucosa of the patient and displace the position thereof (see Fig. 30B or the like). Depending on a case of a second or subsequent stage when the position of the mucosa is to be displaced in stages or a case where the concave hole 70 is formed to have a size appropriate for transplant of the autologous tooth (hereinafter referred to also as the autogenous tooth) 400, it may also be possible to increase a diameter of the end portion 63 and advance the procedure on the patient. In the present embodiment, a plurality of types of the bar portions 62 with the end portions 63 having different diameters are prepared in advance, and the bar portion 62 is replaced to be able to change the diameter of the end portion 63. At least one of the plurality of bar portions 62 has the end portion 63 having a diameter larger than a diameter of an end portion of an implant-dedicated osteotome. The diameter of the end portion 63 of the bar portion 62 is, e.g., 5 mm or more. It can be said that the bar portion 62 with the end portion 63 having a size (diameter) as described above can appropriately be used particularly for transplant of the autogenous tooth 400, and has not been used in a conventional implant osteotome.

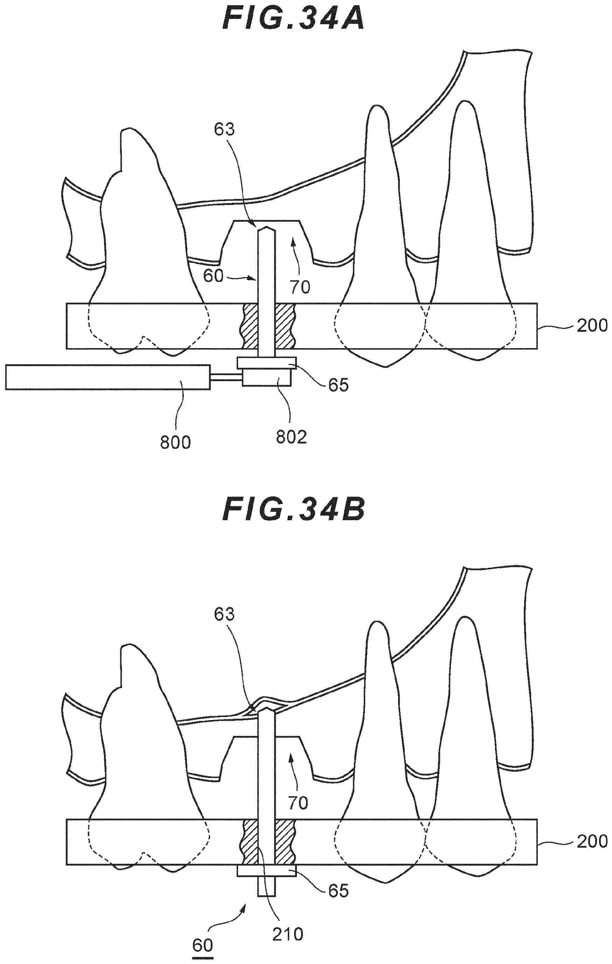

The guided portion 64 is a portion to be guided along the guide hole 210 of the surgical guide 200, and is formed in a portion of the body portion 61 according to a size and a shape of the guide hole 210 (see Fig. 30B or the like). As has been described heretofore, the surgical guide 200 is provided according to a surface shape of the treatment area of the patient, and is formed so as to offset a reference position serving as a guide for a depth during a treatment by a predetermined amount. In the present embodiment, by providing such a structure in which the osteotome 60 is moved by guiding the guided portion 64 along the guide hole 210 of the surgical guide 200, variations which may occur during embedment in the treatment area due to a skill differing from one dentist to another, a condition of the treatment area differing from one patient to another, or the like are reduced to be able to improve reproducibility and accuracy (see Fig. 34A and the like).

The stopper portion 65 is a portion formed so as to come into contact with the surgical guide 200 and restrict a movable range of the osteotome 60. In the present embodiment, a proximal end side of the guided portion 64 described above is formed into a stepped shape having a diameter larger than that of the guided portion 64, and the stepped shape portion is caused to function as the stopper portion 65 (see Fig. 30B and the like).

The display window 66 is formed in the body portion 61 so as to allow information in the information presentation unit 62b provided in the bar portion 62 to be visually recognizable (see Fig. 30B and the like). For example, in the present embodiment, what is obtained by cutting out a portion of the body portion 61 is used as the display window 66 so as to allow the information (such as a numerical value) in the information presentation unit 62b of the bar portion 62 attached to the inside thereof to be visually recognizable (see Fig. 30B and the like).

The screw 67 is a member to be used as an engagement tool for engaging the bar portion 62 with a predetermined position on the body portion 61. By tightening the screw 67 provided in the body portion 61 and engaging a distal end thereof with the engagement grooves 62a of the bar portion 62, it is possible to provide a state where the bar portion 62 is engaged with the predetermined position. Meanwhile, by rotating the screw 67 backward and loosening the screw 67, the engaged state of the bar portion 62 is eliminated, and the bar portion 62 can be slid in a longitudinal direction. In addition, in the osteotome 60 in the present embodiment, the two screws 67 are arranged such that, e.g., the bar portion 62 is held therebetween (see Fig. 31). By arranging the at least two screws 67, even if one of the screws is loosened, it is possible to consider safety by avoiding unexpected movement of the bar portion 62 during the procedure. In the present embodiment, the engagement grooves 62a are provided at least on both sides of the bar portion 62 to allow both of the two screws 67 arranged such that the bar portion 62 is held therebetween to be engaged with the engagement grooves 62a. These two screws 67 may also be placed by being displaced in position along the longitudinal direction (see Fig. 31).

The osteotome 60 provided with a structure as described above in the present embodiment has advantages as shown below.
·Since the entire length is variable, when, e.g., the procedure is performed on the treatment area in, e.g., the upper jaw of the patient, it is possible to adjust the entire length so as to prevent a portion of the osteotome 60 from hitting the lower jaw.
·From the display window 66, the information in the information presentation unit 62b provided in the bar portion 62 can be recognized at a glance.
·By moving the osteotome 60 while guiding the guided portion 64 in the guide hole 210 of the surgical guide 200, it is possible to implement the concave hole 70 at a position and a depth and in a direction exactly according to the plan.

Note that the osteotome 60 described above is only a preferred example. For example, in a case where the osteotome 60 with the end portion 63 having a size (diameter) exceeding a predetermined value is applied for transplant of the autogenous tooth 400, when the position of the mucosa is to be displaced so-called freehand without using the surgical guide 200 described above, it is also possible to use the osteotome 60 that does not include the guided portion 64. Alternatively, it may also be possible to adopt the bar portion 62 of a screw type structure, i.e., having a structure in which helical unevenness is formed around the bar portion 62 and provide a structure in which the unevenness is screwed to helical unevenness formed in an inner wall of the hollow portion 61a of the body portion 61, though not particularly shown. By turning the bar portion 62, it is possible to move the bar portion 62 along a center axis direction thereof and change an amount of protrusion from the body portion 61.

### (Fourth Embodiment)

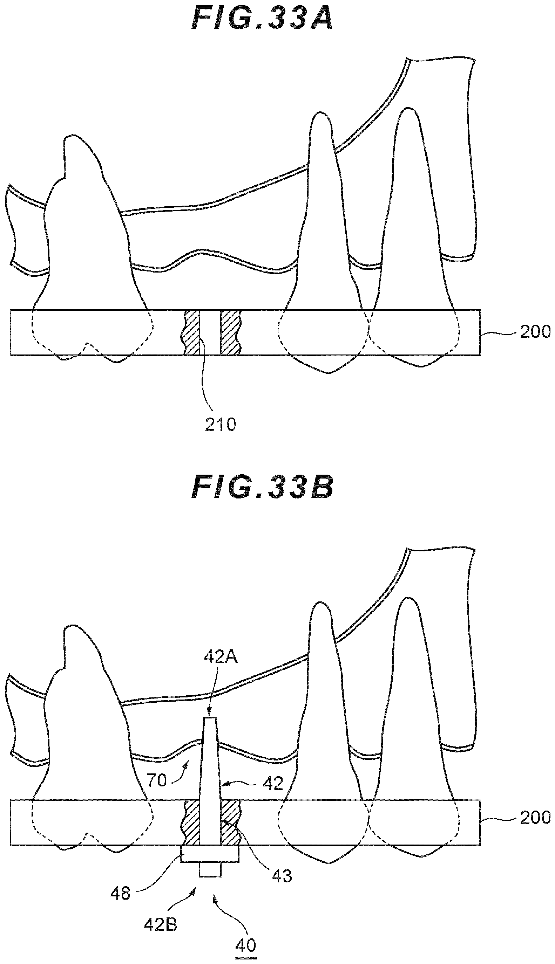

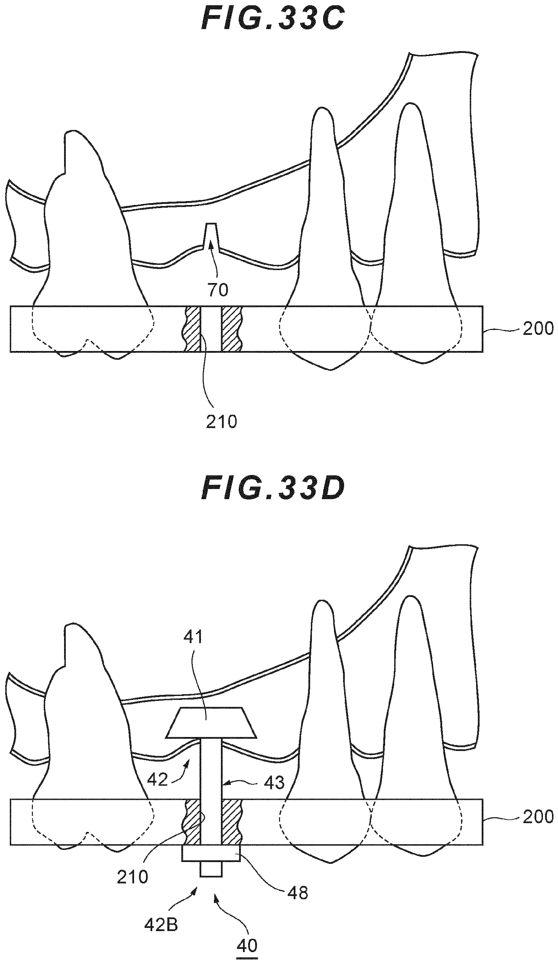

A fourth embodiment of the present invention relates to the hole formation instrument 40 for transplant. The hole formation instrument 40 is an instrument having a drill portion 41 which advances from a surface layer of a treatment area in a jaw region of a patient to form a hole in which a portion of an embedded body is to be embedded (see Figs. 33B and 33D and the like). The hole formation instrument 40 in the present embodiment has the drill portion 41 having a diameter appropriate for transplant of the autogenous tooth 400 (see Fig. 33D and the like). A diameter of the drill portion 41 appropriate for the transplant of the autogenous tooth 400 is at least larger than a drill diameter of a hole formation instrument for implant, which is specifically as large as at least 5 mm or more. It can be said that the hole formation instrument 40 including the drill portion 41 having such a diameter as described above can appropriately be used particularly for the transplant of the autogenous tooth 400, and has not been used as a conventional hole formation instrument for implant. Note that, in Figs. 33B and the like, a reference sign 42 denotes a body, a reference sign 42A denotes a distal end of the body 42, and a reference sign 42B denotes a proximal end of the body 42.

Additionally, in a portion of the hole formation instrument 40 in the present embodiment, the guided portion 43 and the stopper 48 are formed. The guided portion 43 is formed so as to be guided along the guide hole 210 of the surgical guide 200 and, in the present embodiment, by using a structure in which the hole formation instrument 40 is moved, while the guided portion 43 is guided along the guide hole 210 of the surgical guide 200 described above, it is possible to reduce variations which may occur during embedment in the treatment area due to a skill differing from one dentist to another, a condition of the treatment area differing from one patient to another, or the like and improve reproducibility and accuracy (see Fig. 33D and the like).

### (Example of Procedure Using Osteotome in Third Embodiment, Hole Formation Instrument in Fourth Embodiment, etc.)

A sequential flow of the procedure by the dentist when the concave hole 70 for the transplant of the autogenous tooth 400 is to be formed in the jaw region of the patient by using the hole formation instrument 40 used in the present embodiment, the osteotome 60 used in the third embodiment described above, and a water flow tube 700, which is shown in the view, will be described (see Figs. 33A to 35E and the like).

When the surgical guide 200 is fitted to the jaw region (e.g., the upper jaw) of the patient (see Fig. 33A), the concave hole 70 is formed by using the hole formation instrument 40 (see Fig. 33B). At this time, by moving the hole formation instrument 40 to embed the distal end thereof in the guide hole 210 of the surgical guide 200, while guiding the guided portion 43, it becomes easier to form the concave hole 70 at a position and a depth and in a direction exactly according to the plan. When the stopper 48 comes into contact with the surgical guide 200 (see 33B), the hole formation instrument 40 is pulled out (see Fig. 33C).

Then, using the hole formation instrument 40 having the drill portion 41 having a larger diameter, the larger concave hole 70 is formed (see Fig. 33D). Note that, by adopting the drill portion (drill head) 41 detachable from the shank 44, inserting the shank 44 through the guide hole 210, and then attaching the drill portion 41 to the distal end of the shank 44, it is possible to implement a state illustrated in Fig. 33D (state where the drill portion 41 larger than an inner diameter of the guide hole 210 is present between the surgical guide 200 and the jaw region of the patient), though details thereof are not shown in the figure.

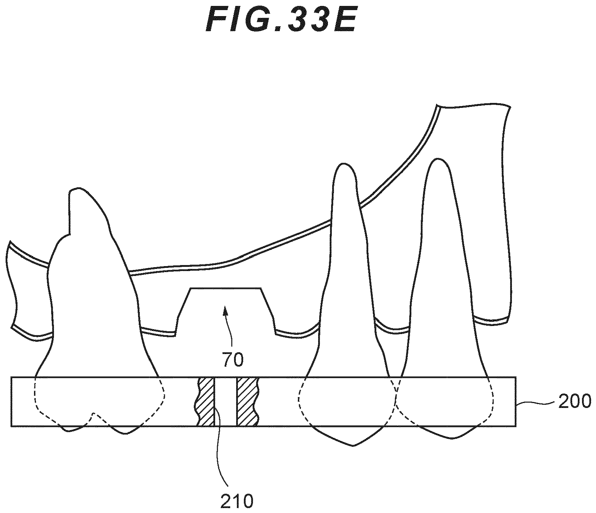

When the concave hole 70 having a size appropriate for the transplant of the autogenous tooth 400 is formed, the surgical guide 200 and the hole formation instrument 40 are detached, and then the surgical guide 200 is attached again to the jaw region of the patient (see Fig. 33E). Note that, in the present embodiment, an example in which the drill diameters of two sizes are used to form the concave hole 70 of a size appropriate for the transplant of the autogenous tooth 400 by a so-called two-stage procedure has been shown (see Figs. 33B and 33D), but this is a description of a simplified example and, needless to say, the concave hole 70 of a size appropriate for the transplant of the autogenous tooth 400 may also be formed by a procedure including three or more stages, with drill diameters of three or more sizes being employed.

Subsequently, using the osteotome 60 or the like, a position of a mucosa behind the concave hole 70 is displaced. Here, the bar portion 62 of the osteotome 60 is inserted first from the end portion 63 side thereof into the guide hole 210 of the surgical guide 200, and the proximal end side of the body portion 61 is repeatedly hit with a head 802 of a hammer 800 to gradually displace the position of the mucosa with the end portion 63 (see Fig. 34A). The procedure (displacement of the position of the mucosa) using the osteotome 60 is continued until the stopper portion 65 comes into contact with the surgical guide 200 (see Fig. 34B). Note that Fig. 34A or the like illustrates the osteotome 60 in a simplified manner, but this simply illustrates only a basic structure, and is not intended to illustrate an osteotome having a different structure. In addition, in Figs. 34A and 34B, a shaft portion (bar portion) of the osteotome 60 has different lengths, but this represents that the amount of protrusion to the end portion 63 and the entire length of the osteotome 60 are appropriately changed by sliding the bar portion 62 in the longitudinal direction along the hollow portion 61a.

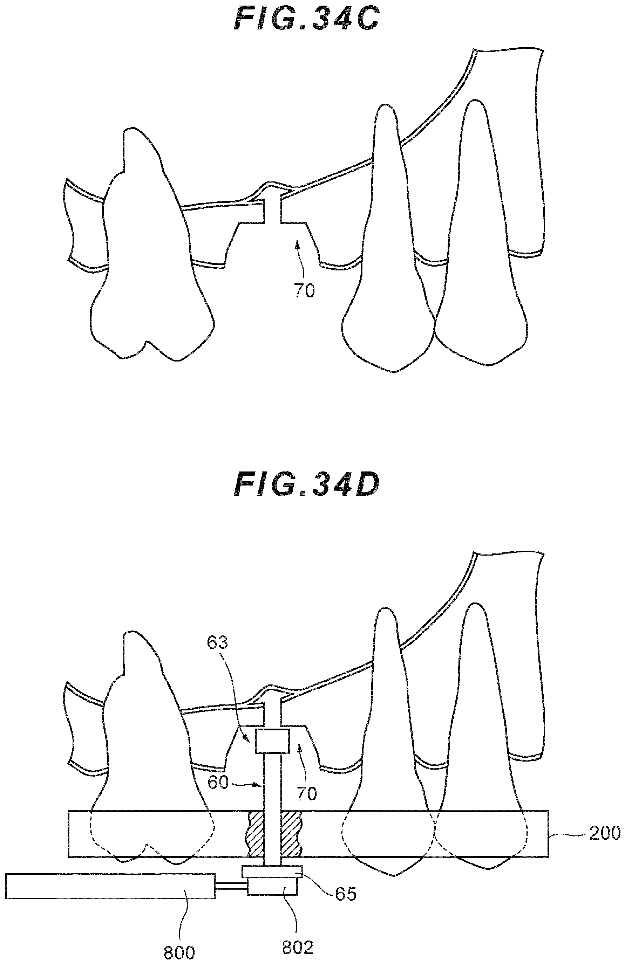

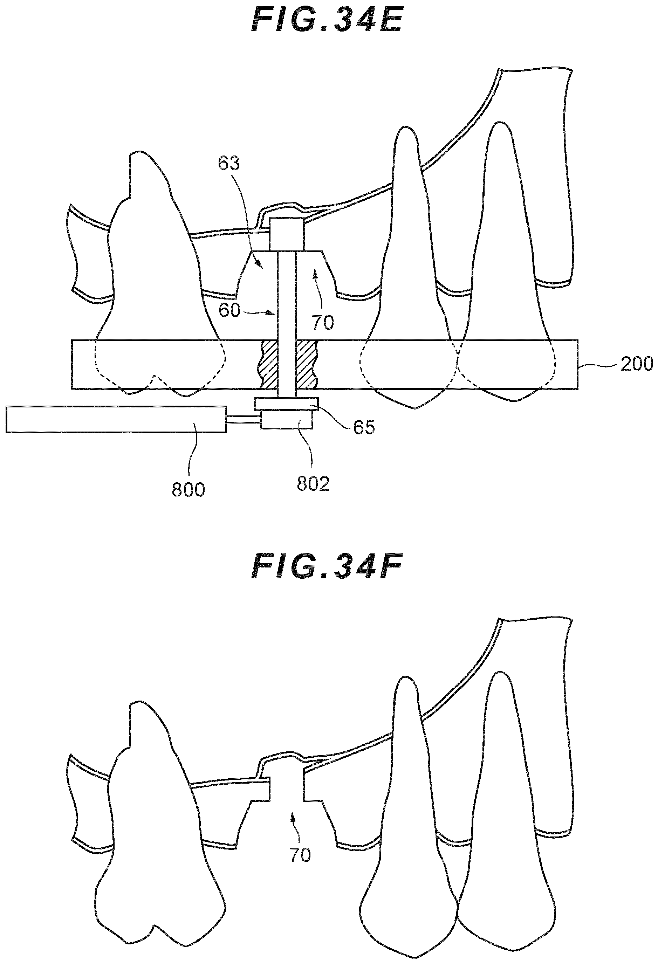

When the stopper portion 65 comes into contact with the surgical guide 200 (see Fig. 34B), the osteotome 60 and the surgical guide 200 are temporarily detached (see Fig. 34C). Then, using the osteotome 60 with the end portion 63 having a larger diameter, the position of the mucosa behind the concave hole 70 is displaced in a wider range (see Figs. 34D and 34E). Note that, when the bar portion 62 is replaced, the proximal end side of the bar portion 62 is inserted through the guide hole 210 of the surgical guide 200, and then the bar portion 62 is attached to the body portion 61, it is possible to implement a state (state where the end portion 63 larger than the inner diameter of the guide hole 210 is present between the surgical guide 200 and the jaw region of the patient) illustrated in Fig. 34D.

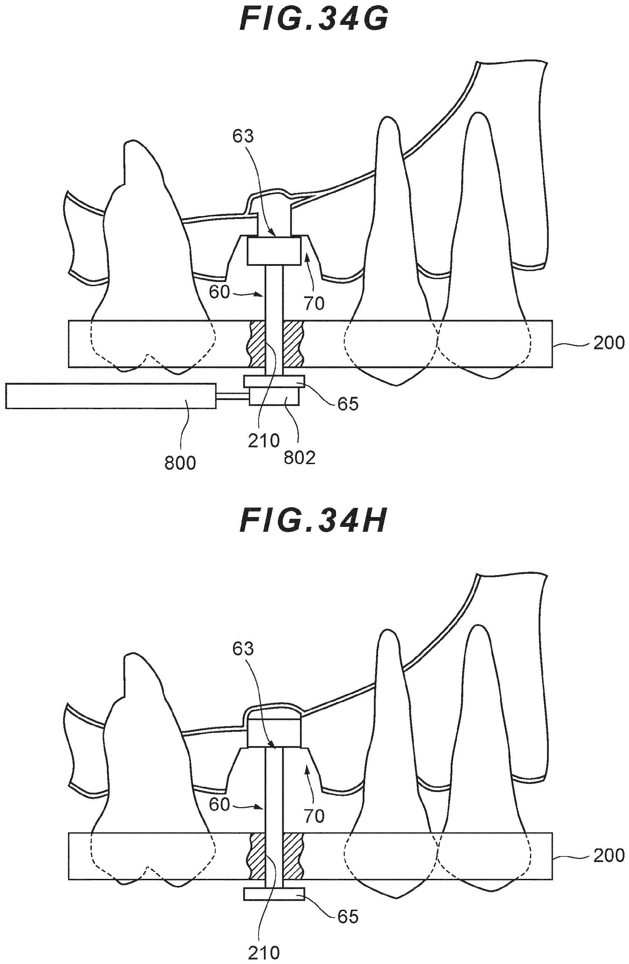

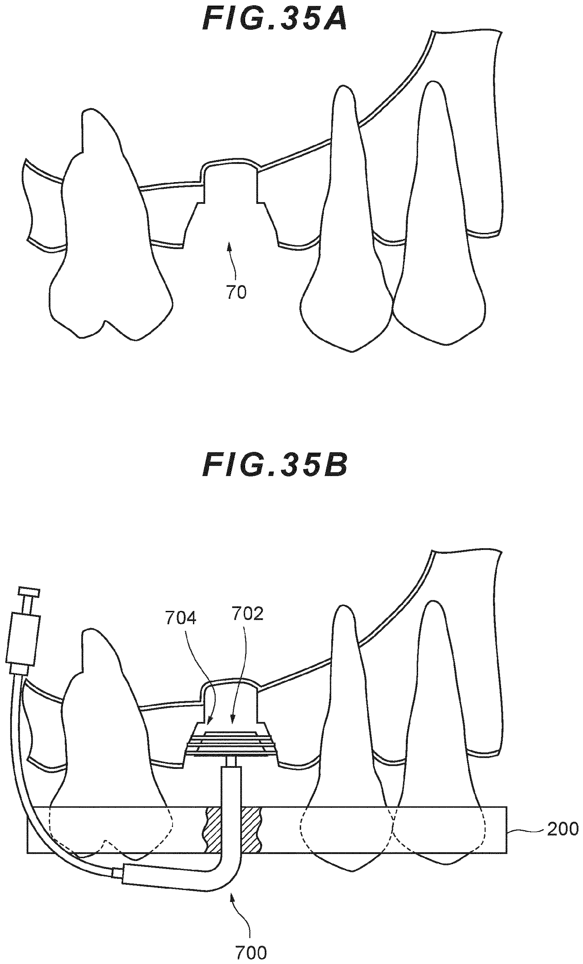

Then, the surgical guide 200 and the osteotome 60 are detached (see Fig. 34F) and, using the osteotome 60 with the end portion 63 having an even larger diameter, the position of the mucosa behind the concave hole 70 is displaced in a wider range (see Figs. 34G and 34H). When the position of the mucosa behind the concave hole 70 is displaced to an extent appropriate for the transplant of the autogenous tooth 400, the surgical guide 200 and the osteotome 60 are detached (see Fig. 35A). Note that, in the present example, an example in which the osteotome 60 with the end portions 73 of three different sizes is used to displace the position of the mucosa by a so-called three-stage procedure is shown (see Figs. 34B, 34D, and 34H and the like), but this is a description of a simplified example and, needless to say, it may also be possible to adopt the osteotome 60 with the end portion 63 of four or more sizes and displace the position of the mucosa by a procedure including four or more stages.

Subsequently, the surgical guide 200 with the water flow tube 700 attached thereto is fitted to the jaw region (e.g., the upper jaw) of the patient, and apply a distal end portion 702 of the water flow tube 700 to the concave hole 70 (see Fig. 35B). At this time, it is preferable that a state where a stepped portion 704 formed in the distal end portion 702 is in contact with an inner wall of the concave hole 70 is established (see Fig. 35B). Note that a detailed structure of the water flow tube 700, such as the distal end portion 702 and the stepped portion 704, will be described in the subsequent embodiment.

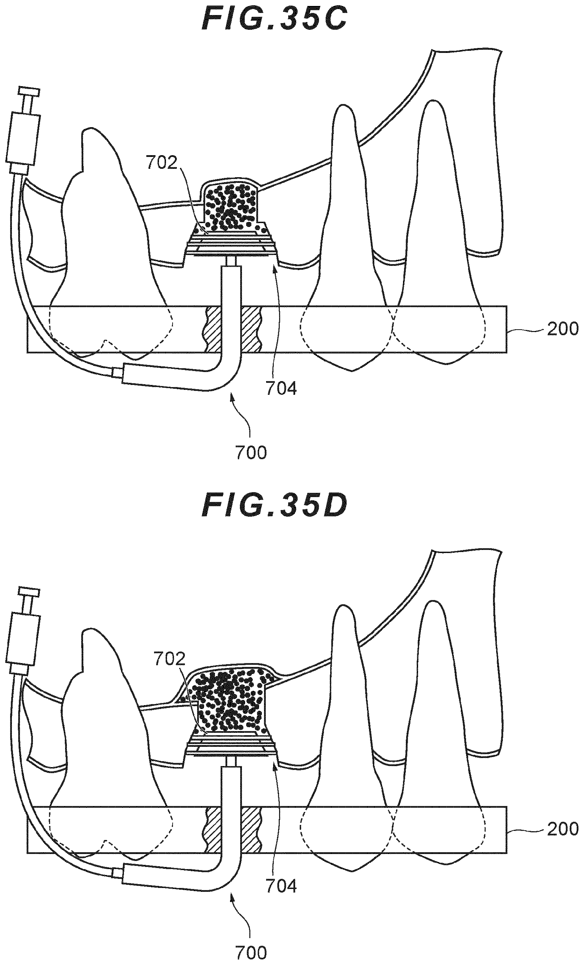

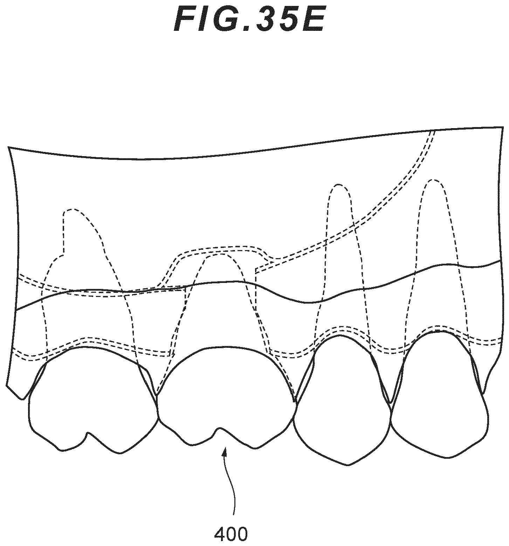

When a state is established in which the distal end portion 702 of the water flow tube 700 is applied to the concave hole 70 (see Fig. 35B), water is jetted from the distal end portion 702 to further displace the position of the mucosa behind the concave hole 70 (Fig. 35C). When the mucosa is displaced into a state appropriate for transplant (see Fig. 35D), the water flow tube 700 and the surgical guide 200 are detached, and the autogenous tooth 400 is transplanted in the concave hole 70 (see Fig. 35E).

### (Fifth Embodiment)

A fifth embodiment of the present invention relates to a test post 410. The test post 410 is a specimen inserted into a hole in a predetermined area to form an image in an image photographed in that state by using a radioactive ray and indicate a position of the hole, which is used for image diagnosis. The hole in the predetermined area mentioned herein is, e.g., a hole formed to allow a portion of an embedded body (the implant body 30 or the autogenous tooth 400) to be embedded in the treatment area of the jaw region of the patient or the guide hole 210 formed in the surgical guide 200 described above.

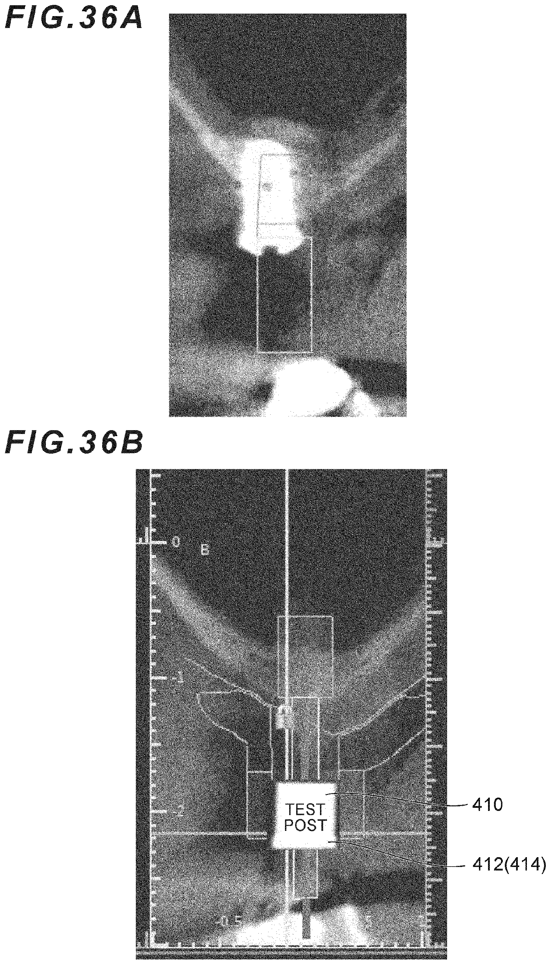

In light of the previous example when a procedure as has been described heretofore is performed, displacement of the position of the embedded body (e.g., a white portion in the vicinity of a center in Fig. 36A) from a scheduled target position (a frame portion in the vicinity of the center in Fig. 36A) may actually occur (see Fig. 36A). Considering this, as a reason therefor, mismatching, distortion of an impression material, gypsum swelling, scan distortion, distortion during CT data conversion to three dimensions, or the like can be considered but, in either case, in light of the fact that such a situation occurs with a reasonable frequency, it is important to timely check whether or not the procedure is proceeding or can be advanced exactly according to the plan. The test post 410 according to the present embodiment that has been devised in view of such a real situation and circumstances allows timely checking, as described above.

A specific example of the test post 410 will be described. The test post 410 in the present embodiment is formed of a material that forms an image in an image photographed by using a radioactive ray, e.g., a resin material or a ceramic material such as zirconium (see Fig. 36B). A specific size of the test post 410 is not particularly limited as long as the test post 410 is formed in a size according to the concave hole 70 in the treatment area as a target of positional detection or the guide hole 210 of the surgical guide 200.

In the present embodiment, the test post 410 formed with a flange-shaped stepped portion 412 such that an outer diameter changes midway is adopted (see Fig. 36B). It is also possible to allow a portion of an outer diameter portion 414 having a maximum diameter of the test post 410 (in the test post 410 illustrated in Fig. 36B, the portion of the stepped portion 412 corresponds thereto) to have a diameter larger than the inner diameter of the guide hole 210 of the surgical guide 200. In such a case, the outer diameter portion 414 functions as a stopper that comes into contact with the surgical guide 200, and it is possible to capture an image in a state where the surgical guide 200 is positioned at a predetermined position of the guide hole 210.

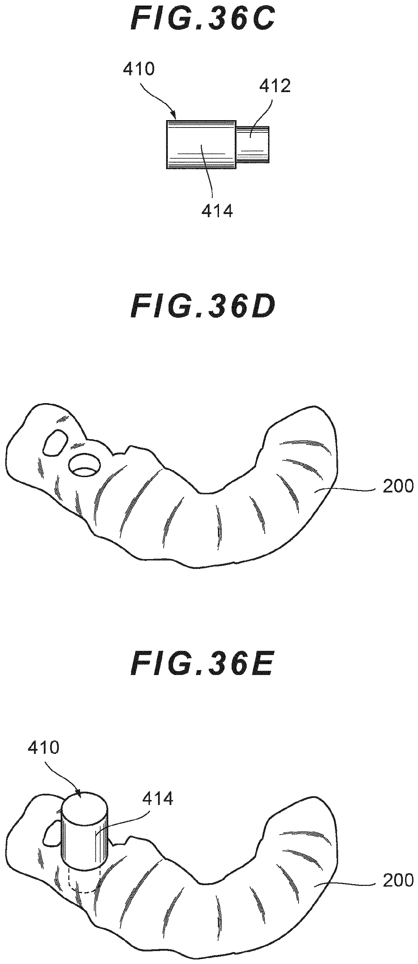

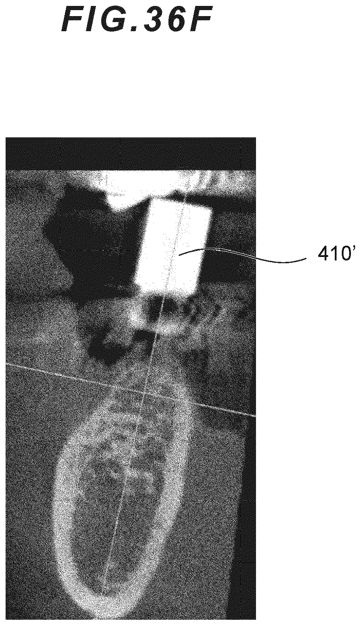

Another use mode of the test post 410 will be described herein (see Figs. 36C to 36F). In the present example, the test post 410 is attached (see Fig. 36E) to the surgical guide 200 (see Fig. 36D) actually produced on the basis of the sixth three-dimensional image (three-dimensional image of the surgical guide 200) 56 of the auxiliary instrument, the surgical guide 200 is actually attached to the patient exactly in a state where the test post 410 is attached, and a CT image is photographed (see Fig. 36F). By checking a position of the test post (denoted by a reference sign 410') appearing in a CT image, it is possible to check a discrepancy (difference or deviation) from design of the actually produced surgical guide 200. As the test post 410 for such an application, the test post 410 in which the portion of the outer diameter portion 414 having the maximum diameter has a relatively large axial direction length can be adopted (see Fig. 36C).

### (Sixth Embodiment)

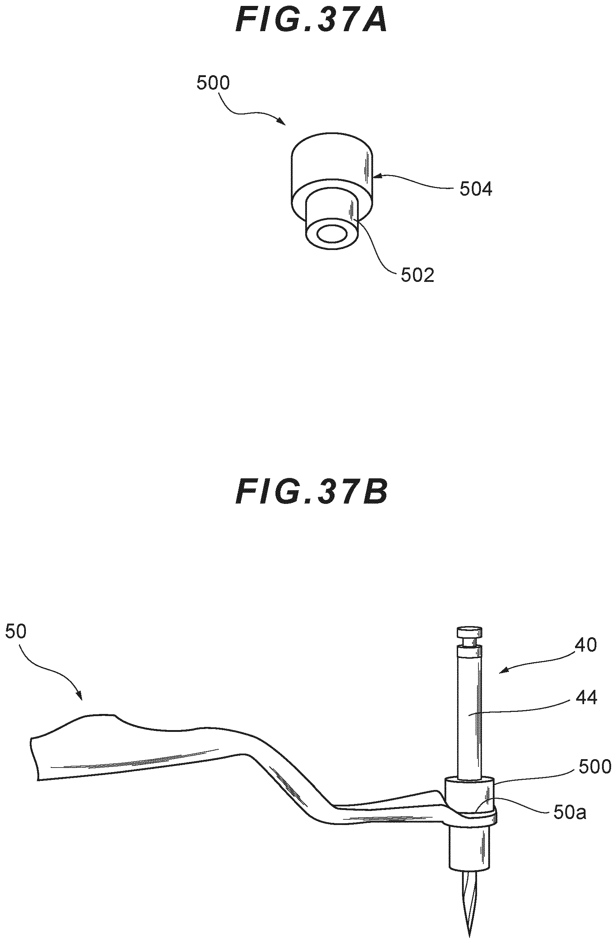

A sixth embodiment of the present invention relates to a stopper extension. A stopper extension 500 is configured as a member that is inserted into the guide hole 210 of the surgical guide 200 to extend a guide length (see Figs. 37A to 37C).

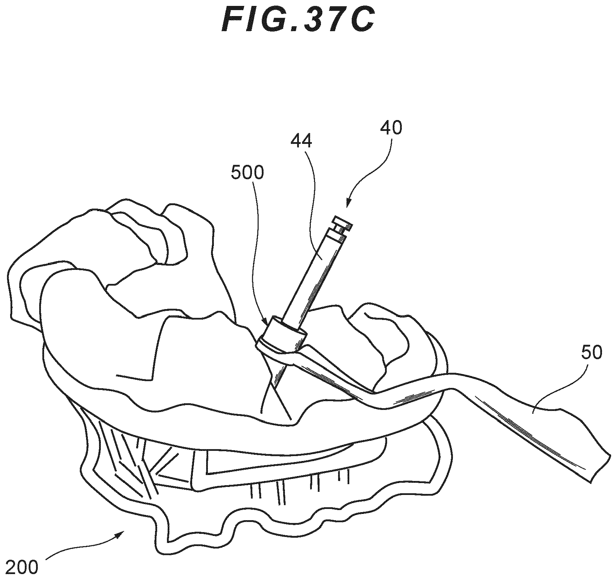

The stopper extension 500 in the present embodiment is made of a cylindrically formed member having a stepped shape and made of, e.g., a resin (see Fig. 37A). The stopper extension 500 is formed such that an outer diameter thereof has a size according to an inner diameter of a guide hole 50a in the distal end of the handle 50, and can be attached to the handle 50 in such a manner that a portion (portion except for the stepped portion 502) thereof is inserted into the guide hole 50a (see Fig. 37B). Meanwhile, an inner diameter of the stopper extension 500 is set to have a size according to a diameter of the shank 44 of the hole formation instrument 40 to be used. In addition, an outer diameter portion of the stopper extension 500 that has a large diameter (in the case of the stopper extension 500 in the present embodiment, the stepped portion 502 corresponds thereto) has a predetermined size, e.g., a diameter larger than the inner diameter of the guide hole 210 of the surgical guide 200 or a diameter larger than the inner diameter of the guide hole 50a in the distal end of the handle 50 (see Figs. 37B and 37C).

With the stopper extension 500 thus configured, the guide length in an axial direction can be set longer than when the hole formation instrument 40 or the like is guided only with the handle 50. Therefore, during the procedure using the hole formation instrument 40, it is possible to reduce lateral movement of the hole formation instrument 40 and improve a linear directivity.

In addition, the stopper extension 500 can function not only as a member that extends the guide length, but also as a member for changing a position with which the stopper (such as, e.g., the stopper 48 of the hole formation instrument 40 or the stopper portion 65 of the osteotome 60) comes into contact. By way of example, when a thickness of, e.g., the flange-shaped stepped portion 502 is set to a predetermined value (e.g., 1 mm), it is possible to displace the position with which the stopper comes into contact in units of a given value.

### (Seventh Embodiment)

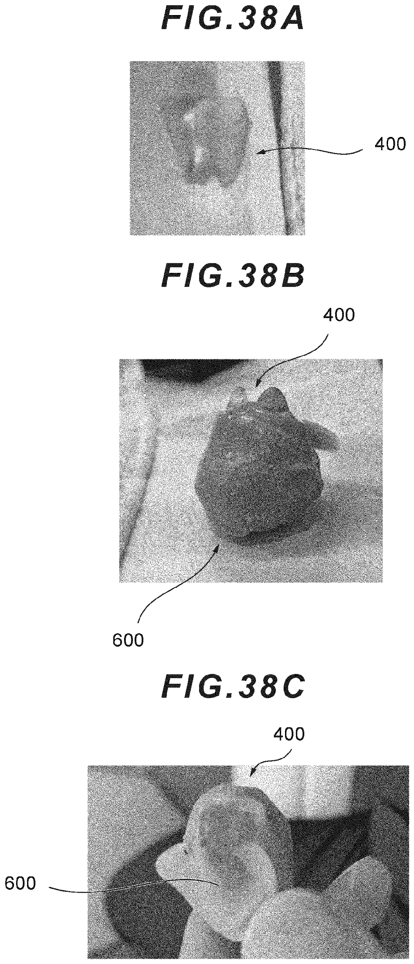

A seventh embodiment of the present invention relates to a periodontal ligament guard. A periodontal ligament guard 600 is a member that partially or entirely covers a portion of the autogenous tooth 400 to be subjected to transplant except for a tooth root to be cut (see Figs. 38A to 38C). By using the periodontal ligament guard 600, it is possible to reduce damage due to an external cause (such as, e.g., heat which may be generated during cutting or a water flow or an oil content used during the cutting) which may be given to a periodontal membrane located around the tooth root when the tooth root is cut. The periodontal ligament guard 600 may also be formed of a low resilience material. With the periodontal ligament guard 600 formed of such a material, when the autogenous tooth 400 in a state covered with the periodontal ligament guard 600 is held between fingers, the periodontal ligament guard 600 is freely deformed into an easy-to-hold shape, and therefore an operation such as cutting of the tooth root is easily performed (see Fig. 38C and the like). From such a viewpoint, the periodontal ligament guard 600 may also be made of silicon or the like. Note that it is also possible to use the periodontal ligament guard 600 by covering the entire autogenous tooth 400 with the periodontal ligament guard 600 and cutting the tooth root together with the periodontal ligament guard 600, though not particularly shown.

### (Eighth Embodiment)

An eighth embodiment of the present invention relates to a water flow tube. A water flow tube 700 is an instrument which is embedded from a surface layer of a treatment area (such as the concave hole 70) in a jaw region of a patient into a deep portion to jet water from a distal end portion 72 toward a position of a mucosa behind the treatment area. In the present embodiment, a diameter of the distal end portion 702 of the water flow tube 700 is set to a size particularly appropriate for the transplant of the autogenous tooth 400, more specifically to a size according to the size of the concave hole 70 which is generally larger than when the implant body 30 is to be embedded (see Fig. 35B and the like). Moreover, in the present embodiment, the distal end portion 702 of the water flow tube 700 is formed with the stepped portion 704 having an outer diameter which stepwise increases with distance from the distal end toward the proximal end (see Fig. 35C and the like). The stepped portion 704 can be formed of, e.g., a multi-stage flange-shaped protruding portion made of, e.g., an elastic member (e.g., rubber material). The stepped portion 704 is elastically deformed appropriately to further improve adhesion to the concave hole 70, and can thereby further improve efficiency when the mucosa is to be displaced with jetted water (see Figs. 35C and 35D and the like).

Note that the present invention is not limited to the embodiments described above, and can variously be modified at an implementation stage within a scope not departing from the gist thereof. The individual embodiments can also be implemented by being combined with each other as appropriate as possible and, in that case, combined effects can be obtained. Moreover, the embodiments described above include the invention at various stages, and an appropriate combination of a plurality of disclosed constituent features allows various inventions to be extracted.

### Reference Signs List

- 10: Pre-procedural verification system for dental treatment plan
- 12: Control device
- 18: Display unit
- 20: Operation unit
- 30: Implant body (embedded body)
- 40: Hole formation instrument
- 41: Drill portion
- 42: Body
- 42A: Distal end of body
- 42B: Proximal end of body
- 43: Guided portion
- 44: Shank
- 46: Sleeve (drill sleeve)
- 48: Stopper
- 48a: End surface of stopper 48 on body 42 side
- 50: Handle
- 50a: Guide hole of handle
- 51: First three-dimensional image data (sixth surface data)
- 52: Second three-dimensional image data (first surface data)
- 53: Third three-dimensional image data (fourth surface data)
- 54: Fourth three-dimensional image data (second surface data)
- 55: Fifth three-dimensional image data (third surface data)
- 56: Sixth three-dimensional image
- 60: Osteotome
- 61: Body portion
- 61a: Hollow portion
- 62: Bar portion
- 62a: Engagement groove
- 62b: Information presentation unit
- 63: End portion
- 64: Guided portion
- 65: Stopper portion
- 66: Display window
- 67: Screw (engagement tool)
- 70: Concave hole
- 100: Model
- 100a: Three-dimensional image data (fifth surface data)
- 110: Model main body
- 110a: Three-dimensional image data
- 120: Target model portion
- 120a: Three-dimensional image data
- 130: Hole portion (through hole)
- 200: Surgical guide (auxiliary instrument)
- 210: Guide hole
- 220: Defining surface
- 310: Lower jaw
- 312: Alveolar bone
- 314: Tooth
- 316: Gum
- 318: Concave hole
- 320: Not-to-be-reached target
- 400: Autologous tooth (autogenous tooth)
- 410: Test post
- 412: Stepped portion
- 414: Outer diameter portion
- 500: Stopper extension
- 502: Stepped portion
- 504: Outer diameter portion
- 600: Periodontal ligament guard
- 700: Water flow tube
- 702: Distal end portion
- 704: Stepped portion
- 800: Hammer
- 802: Head
- H1: Distance from proximal end portion (46b) of drill sleeve to distal end of drill body (42)
- H2: Distance from proximal end portion (46b) of drill sleeve to stopper (48) at distal end
- D1: Length of implant body (30) or autologous tooth
- D2: Value of amount of offset due to use of surgical guide

## Claims

1. An osteotome used as an instrument which is embedded from a surface layer of a treatment area in a jaw region of a patient into a deep portion to apply an impact in expanding or compressing a bone so as to displace a position of a mucosa behind the treatment area, the osteotome comprising:
a body portion corresponding to a main body portion of the osteotome; and
an end portion formed at a distal end of the body portion or a member, which is attached to the body portion, to displace the position of the mucosa,
a diameter of the end portion having a size appropriate for transplant of an autogenous tooth.

2. The osteotome according to claim 1, wherein the diameter of the end portion is larger than a diameter of an end portion of an implant-dedicated osteotome.

3. The osteotome according to claim 2, wherein the diameter of the end portion is 5 mm or more.

4. The osteotome according to claim 3, wherein a guided portion which is guided along a guide hole formed in a surgical guide is formed in a portion of the body portion, the surgical guide being provided according to a surface shape of the treatment area of the patient to offset, by a predetermined amount, a reference position serving as a guide for a depth during a treatment.

5. An osteotome used as an instrument which is embedded, from a proximal end side to a distal end side, in a treatment area in a jaw region of a patient to apply an impact in expanding or compressing a bone so as to displace a position of a mucosa behind the treatment area, the osteotome comprising:
a body portion corresponding to a main body portion of the osteotome; and
an end portion formed at a distal end of the body portion to displace the position of the mucosa,
a guided portion which is guided along a guide hole formed in a surgical guide being formed in a portion of the body portion, the surgical guide being provided according to a surface shape of the treatment area of the patient to offset, by a predetermined amount, a reference position serving as a guide for a depth during a treatment.

6. A hole formation instrument comprising:
a drill portion that is moved from a surface layer of a treatment area in a jaw region of a patient toward a deep portion to form a hole in which a portion of an embedded body is to be embedded,
a diameter of the drill portion having a size appropriate for transplant of an autogenous tooth.

7. The hole formation instrument according to claim 6, wherein the diameter of the drill portion is larger than a diameter of a drill portion when the embedded body is an implant body.

8. The hole formation instrument according to claim 7, wherein the diameter of the drill portion is 5 mm or more.

9. The hole formation instrument according to claim 8, wherein a guided portion which is guided along a guide hole provided in a surgical guide is formed in a portion of the hole formation instrument, the surgical guide being provided according to a surface shape of the treatment area of the patient to offset, by a predetermined amount, a reference position serving as a guide for a height during a treatment.

10. A test post for image diagnosis, formed of a resin material or a ceramic material and inserted into a hole formed in a treatment area in a jaw region of a patient to allow a portion of an embedded body to be embedded therein or into a guide hole formed in a surgical guide to form an image in an image photographed in a state of the insertion by using a radioactive ray and indicate a position of the hole or the guide hole, the surgical guide being provided according to a surface shape of the treatment area of the patient to offset, by a predetermined amount, a reference position serving as a guide for a depth during a treatment.

11. The test post according to claim 10, which has a stepped shape.

12. The test post according to claim 11, which has an outer diameter portion having a diameter larger than an inner diameter of the guide hole of the surgical guide.

13. A stopper extension formed into a cylindrical shape and inserted into a guide hole formed in a surgical guide to extend a guide length, the surgical guide being provided according to a surface shape of a treatment area of a patient to offset, by a predetermined amount, a reference position serving as a guide for a depth during a treatment.

14. The stopper extension according to claim 13, which has a stepped shape.

15. The stopper extension according to claim 13, which has an outer diameter portion having a diameter larger than an inner diameter of the guide hole of the surgical guide.

16. A periodontal ligament guard which partially or entirely covers a portion of an autogenous tooth to be subjected to transplant, except for a tooth root to be cut, to reduce a damage that is possibly given, by an external cause, to a periodontal membrane located around the tooth root when the tooth root is cut.

17. The periodontal ligament guard according to claim 16, which is formed of a low resilience material.

18. The periodontal ligament guard according to claim 16, which is made of silicon.

19. A water flow tube for transplant, which is embedded from a surface layer of a treatment area in a jaw region of a patient toward a deep portion to jet water from a distal end portion thereof toward a position of a mucosa behind the treatment area,
a diameter of the distal end portion having a size appropriate for transplant of an autogenous tooth.

20. The water flow tube according to claim 19, wherein the distal end portion is formed with a stepped portion having an outer diameter increasing stepwise, with a distance from a distal end toward a proximal end gradually increasing.

21. A model for pre-procedural verification of a dental treatment plan, the model comprising:
a model main body formed on the basis of a three-dimensional image of a jaw region of a patient to have the same size and shape as those of at least a portion of each of a tooth of the patient to be treated, a gum thereof, and an alveolar bone thereof covered with the gum and imitate at least a portion of each of the tooth of the patient to be treated, the gum thereof, and the alveolar bone thereof;
a target model portion formed on the basis of the three-dimensional image of the patient to be adjacent to or embedded in the alveolar bone of the treatment target of the patient, imitate a not-to-be-contacted target with which a distal end of a procedure instrument selected from among a plurality of the procedure instruments is to be kept from contact during a treatment, and be provided in the model main body to have the same positional relationship as a positional relationship between each of the tooth of the treatment target of the patient, the gum thereof, and the alveolar bone and the not-to-be-contacted target; and
a hole portion formed, on the basis of the three-dimensional image of the patient, at a position in the model main body imitating the gum and alveolar bone of the treatment target of the patient to imitate a hole which allows an embedded body to be embedded in the treatment target and allow a dentist to visually recognize a positional relationship between the procedure instrument and the target when the procedure instrument in the three-dimensional image of the patient is fitted in the hole in the three-dimensional image and the procedure instrument is inserted into the hole in the three-dimensional image,
a fluid for making a notification of, when the procedure instrument comes into contact with the target model portion during pre-procedural verification, the contact being embedded therein.
